# EUROPEAN PATENT SPECIFICATION

(11) **EP 3 049 096 B1**
(45) Date of publication and mention of the grant of the patent: **12.12.2018**
(21) Application number: 14847855.5
(22) Date of filing: 26.09.2014
(51) Int. Cl.: A61K 38/07, A61K 38/06, A61P 25/00, A61P 3/00

(54) **USE OF AROMATIC-CATIONIC PEPTIDES TO TREAT NIEMANN-PICK DISEASE CHARACTERISED BY CHOLESTEROL-INDUCED MITOCHONDRIAL DYSFUNCTION**
VERWENDUNG VON AROMATISCH-KATIONISCHEN PEPTIDEN ZUR BEHANDLUNG VON NIEMANN-PICK-KRANKHEIT GEKENNZEICHNET DURCH CHOLESTERIN-INDUZIERTER MITOCHONDRIEN-DYSFUNKTION
UTILISATION DE PEPTIDES AROMATIQUES CATIONIQUES POUR LE TRAITEMENT DE LA MALADIE DE NIEMANN-PICK CHARACTERISÉE PAR UN DYSFONCTIONNEMENT MITOCHONDRIAL INDUIT PAR LE CHOLESTÉROL

(30) Priority: 27.09.2013 US 201361883513 P
(43) Date of publication of application: 03.08.2016
(73) Proprietor: Cornell University, Ithaca, New York 14850 (US); Rozenberg, Felix, Ithaca, New York 14850 (US)
(72) Inventor: SZETO, Hazel H., New York, New York 10128 (US); BIRK, Alexander, Bellerose, New York 11426 (US); ROZENBERG, Felix, Ithaca, New York 14850 (US)
(74) Representative: Barker Brettell LLP
(86) International application number: PCT/US2014/057826
(87) International publication number: WO 2015/048522

(56) References cited:
- WO-A1-2013/059071
- WO-A1-2013/086020
- WO-A2-2005/072295
- US-A1- 2011 039 766
- MARY CARMEN VÁZQUEZ ET AL: "Oxidative Stress: A Pathogenic Mechanism for Niemann-Pick Type C Disease", OXIDATIVE MEDICINE AND CELLULAR LONGEVITY, vol. 2012, 1 January 2012 (2012-01-01), pages 1-11, XP055342330, US ISSN: 1942-0900, DOI: 10.1155/2012/205713
- YU ET AL.: 'Altered Cholesterol Metabolism in Niemann-Pick Type C1 Mouse Brains Affects Mitochondrial Function.' J BIOL CHEM vol. 280, 25 March 2005, pages 11731 - 11739, XP055334509

## Description

### CROSS-REFERENCE TO RELATED APPLICATIONS

The present application claims the benefit of and priority to U.S. Application No. 61/883,513, filed on September 27, 2013.

### TECHNICAL FIELD

The present technology relates generally to aromatic-cationic peptide compositions and methods of use in treating, preventing or ameliorating cholesterol-induced mitochondrial dysfunction.

### BACKGROUND

The following description is provided to assist the understanding of the reader.

High cholesterol contributes to a significant portion of deaths around the globe. In recent years, mitochondrial dysfunction has been associated with a plethora of different diseases ranging from illnesses like cancer, Parkinson's disease, diabetes, and Alzheimer's (Kiebish *et al.,* 2008; Montero *et al.,* 2010; Paradies *et al.,* 2009). Mitochondrial dysfunction and high cholesterol have been shown to have a variety of deleterious effects on cells.

### SUMMARY

In one aspect, the present invention provides an aromatic-cationic peptide or a pharmaceutically acceptable salt thereof for use in treating a subject suffering from Niemann-Pick Disease characterized by cholesterol-induced mitochondrial dysfunction wherein the aromatic cationic peptide comprises one or more peptides selected from the group consisting of 2',6'-Dmt-D-Arg-Phe-Lys-NH₂ (SS-02), Phe-D-Arg-Phe-Lys-NH₂ (SS-20), and D-Arg-2',6'-Dmt-Lys-Phe-NH₂ (SS-31). In certain embodiments of the method, the aromatic-cationic peptide comprises D-Arg-2',6'-Dmt-Lys-Phe-NH₂ (SS-31). In some embodiments, the salt is an acetate salt, tartrate salt or trifluoroacetate salt.

In some embodiments of the method, treatment comprises reducing or ameliorating one or more symptoms of Niemann-Pick Disease, wherein the symptoms of Niemann-Pick Disease are one or more symptoms selected from the group consisting of: difficulty moving limbs (dystonia), enlargement of the spleen and liver (hepatosplenomegaly), dementia, slurred, irregular speech (dysarthria), discoordinated swallowing (dysphagia), sudden loss of muscle tone which may lead to falls (cataplexy), tremors, difficulties moving the eyes up and down (vertical supranuclear gaze palsy), ataxia, reduced appetite, abdominal distension, thrombocytopenia, jaundice, pain, enlarged bone marrow cavities, thinned cortical bone, distortion of the hip bone, seizures, cherry red spot in the eye, and sleep related disorders.

In some embodiments of the method, the aromatic-cationic peptide is administered orally, parenterally, intravenously, subcutaneously, transdermally, topically or by inhalation.

The disclosure also describes a method for reducing cardiolipin oxidation in a subject suffering from a disease characterized by cholesterol-induced mitochondrial dysfunction comprising administering an effective amount of an aromatic-cationic peptide or a pharmaceutically acceptable salt thereof to the subject. In some embodiments, the salt is an acetate salt, tartrate salt or trifluoroacetate salt.

In some embodiments of the method, the aromatic cationic peptide comprises one or more peptides selected from the group consisting of 2',6'-Dmt-D-Arg-Phe-Lys-NH₂ (SS-02), Phe-D-Arg-Phe-Lys-NH₂ (SS-20), and D-Arg-2',6'-Dmt-Lys-Phe-NH₂ (SS-31). In certain embodiments of the method, the aromatic-cationic peptide comprises D-Arg-2',6'-Dmt-Lys-Phe-NH₂ (SS-31).

In some embodiments of the method, the disease is Niemann-Pick Disease.

In some embodiments of the method, treatment comprises reducing or ameliorating one or more symptoms of Niemann-Pick Disease, wherein the symptoms of Niemann-Pick Disease are one or more symptoms selected from the group consisting of: difficulty moving limbs (dystonia), enlargement of the spleen and liver (hepatosplenomegaly), dementia, slurred, irregular speech (dysarthria), discoordinated swallowing (dysphagia), sudden loss of muscle tone which may lead to falls (cataplexy), tremors, difficulties moving the eyes up and down (vertical supranuclear gaze palsy), ataxia, reduced appetite, abdominal distension, thrombocytopenia, jaundice, pain, enlarged bone marrow cavities, thinned cortical bone, distortion of the hip bone, seizures, cherry red spot in the eye, and sleep related disorders.

In some embodiments of the method, the aromatic-cationic peptide is administered orally, parenterally, intravenously, subcutaneously, transdermally, topically or by inhalation.

The disclosure also describes a method for reducing cholesterol-induced cytochrome c peroxidase activity in a subject suffering from a disease characterized by cholesterol-induced mitochondrial dysfunction comprising: administering an effective amount of an aromatic-cationic peptide or a pharmaceutically acceptable salt thereof to the subject.

In some embodiments of the method, the aromatic cationic peptide comprises one or more peptides selected from the group consisting of 2',6'-Dmt-D-Arg-Phe-Lys-NH₂ (SS-02), Phe-D-Arg-Phe-Lys-NH₂ (SS-20), and D-Arg-2',6'-Dmt-Lys-Phe-NH₂ (SS-31).

In some embodiments of the method, the salt is an acetate salt, tartrate salt, or trifluoroacetate salt.

In some embodiments of the method, the aromatic-cationic peptide is administered orally, parenterally, intravenously, subcutaneously, transdermally, topically or by inhalation.

In some embodiments of the method, the disease is Niemann-Pick Disease.

In some embodiments of the method, treatment comprises reducing or ameliorating one or more symptoms of Niemann-Pick Disease, wherein the symptoms of difficulty moving limbs (dystonia), enlargement of the spleen and liver (hepatosplenomegaly), dementia, slurred, irregular speech (dysarthria), discoordinated swallowing (dysphagia), sudden loss of muscle tone which may lead to falls (cataplexy), tremors, difficulties moving the eyes up and down (vertical supranuclear gaze palsy), ataxia, reduced appetite, abdominal distension, thrombocytopenia, jaundice, pain, enlarged bone marrow cavities, thinned cortical bone, distortion of the hip bone, seizures, cherry red spot in the eye, and sleep related disorders.

The disclosure further describes a method for protecting cytochrome c from cholesterol-induced structural damage in a subject suffering from a disease characterized by cholesterol-induced mitochondrial dysfunction comprising administering an effective amount of an aromatic-cationic peptide or a pharmaceutically acceptable salt thereof to the subject.

In some embodiments of the method, the aromatic cationic peptide comprises one or more peptides selected from the group consisting of 2',6'-Dmt-D-Arg-Phe-Lys-NH₂ (SS-02), Phe-D-Arg-Phe-Lys-NH₂ (SS-20), and D-Arg-2',6'-Dmt-Lys-Phe-NH₂ (SS-31).

In some embodiments of the method, the salt is an acetate salt, tartrate salt, or trifluoroacetate salt.

In some embodiments of the method, the aromatic-cationic peptide is administered orally, parenterally, intravenously, subcutaneously, transdermally, topically or by inhalation.

In some embodiments of the method, the disease is Niemann-Pick Disease.

In some embodiments of the method, treatment comprises reducing or ameliorating one or more symptoms of Niemann-Pick Disease, wherein the symptoms of difficulty moving limbs (dystonia), enlargement of the spleen and liver (hepatosplenomegaly), dementia, slurred, irregular speech (dysarthria), discoordinated swallowing (dysphagia), sudden loss of muscle tone which may lead to falls (cataplexy), tremors, difficulties moving the eyes up and down (vertical supranuclear gaze palsy), ataxia, reduced appetite, abdominal distension, thrombocytopenia, jaundice, pain, enlarged bone marrow cavities, thinned cortical bone, distortion of the hip bone, seizures, cherry red spot in the eye, and sleep related disorders.

### BRIEF DESCRIPTION OF THE FIGURES

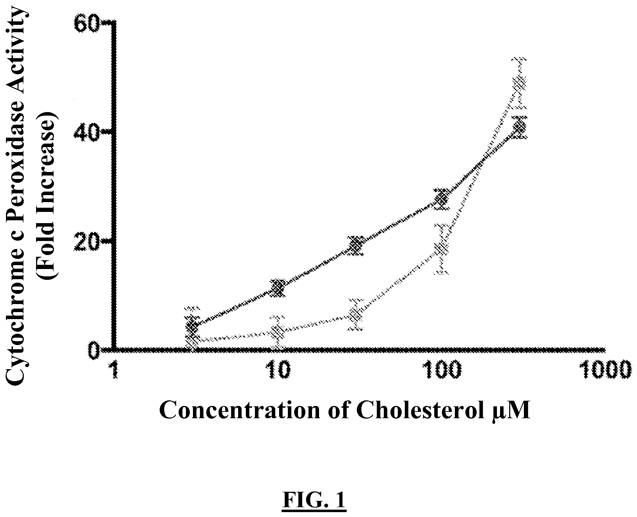

FIG. 1 shows that cholesterol dose-dependently increases cytochrome c peroxidase activity. The increase in cytochrome c peroxidase activity is due to increasing concentrations of cholesterol (ranging from 0 to 300 µM cholesterol) in the form of either free cholesterol (represented by the black circles) or chol-POPC (represented by the gray squares) to 2 µM cytochrome c. Cytochrome c peroxidase activity was measured using the fluorescence of 50 µM Amplex Red Reagent in the presence of 10 µM H₂O₂.

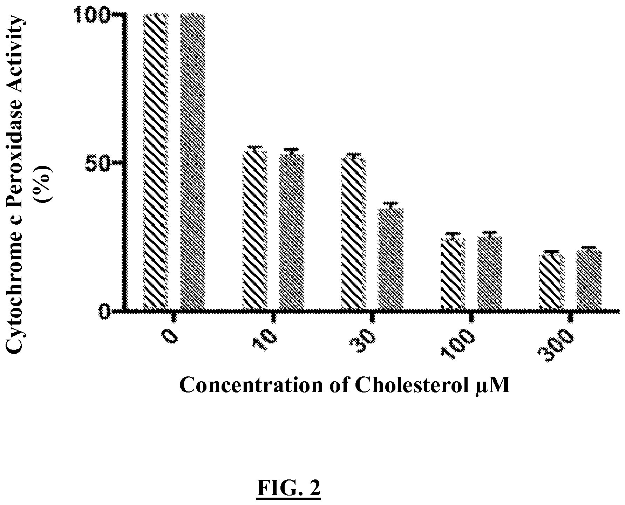

FIG. 2 shows that the D-Arg-2',6'-Dmt-Lys-Phe-NH₂ (SS-31) peptide (represented by the solid bars) and Phe-D-Arg-Phe-Lys-NH₂ peptide (SS-20) (represented by the dashed bars) inhibit cholesterol-induced cytochrome c peroxidase activity in a dose-dependent manner. The addition of various concentrations of D-Arg-2',6'-Dmt-Lys-Phe-NH₂ (SS-31) and Phe-D-Arg-Phe-Lys-NH₂ (SS-20) ranging from 0 µM to 300 µM significantly reduces the effect of 300 µM cholesterol with respect to inducing the peroxidase activity of 2 µM cytochrome c. Cytochrome c peroxidase activity was measured using the fluorescence of 50 µM Amplex Red reagent in the presence of 10 µM H₂O₂.

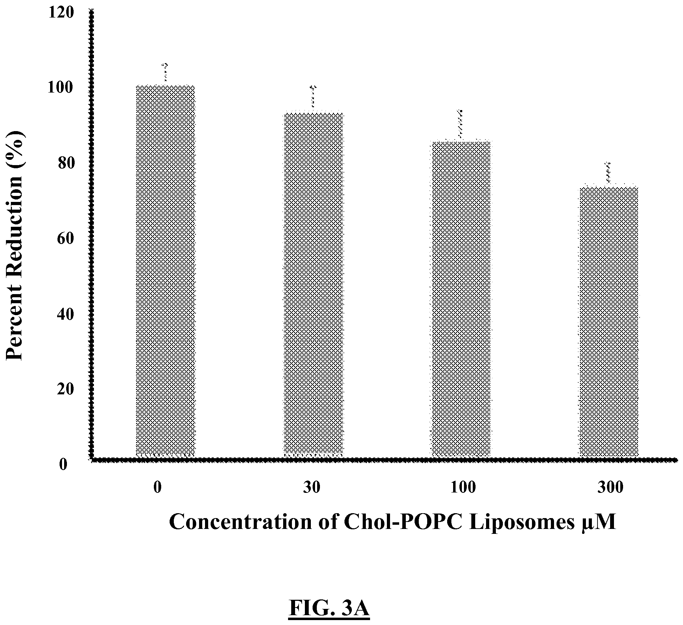

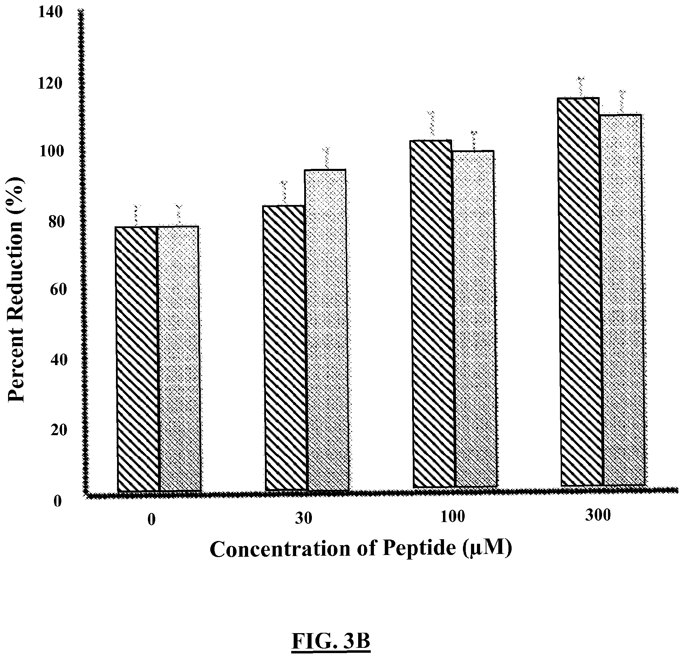

FIG. 3 shows that treatment with D-Arg-2',6'-Dmt-Lys-Phe-NH₂ (SS-31) and Phe-D-Arg-Phe-Lys-NH₂ (SS-20) mitigates the effects of cholesterol on cytochrome c reduction. FIG. 3A shows that addition of increasing amounts of cholesterol exhibits a decrease in the reduction capacity of cytochrome c relative to reduction of cytochrome c without cholesterol. Chol-POPC liposomes were added to 20 µM cytochrome c in 20 mM Hepes buffer pH 7.4 and 50 µM Vitamin C. FIG. 3B shows the effects of pretreating 100 µM chol-POPC liposomes with 20 µM cytochrome c (in 20 mM Hepes buffer pH 7.4 and 50 µM vitamin C) and different concentrations of either D-Arg-2',6'-Dmt-Lys-Phe-NH₂ (SS-31) (represented by the solid bars) and Phe-D-Arg-Phe-Lys-NH₂ (SS-20) (represented by the dashed bars) ranging from no peptide to 300 µM peptide. The results demonstrated a substantial increase in cytochrome c reduction capacity as the peptide concentrations increased. D-Arg-2',6'-Dmt-Lys-Phe-NH₂ (SS-31) and Phe-D-Arg-Phe-Lys-NH₂ (SS-20) returned the reduction capability of cytochrome c back to its initial state prior to treatment with 100 µM chol-POPC liposomes.

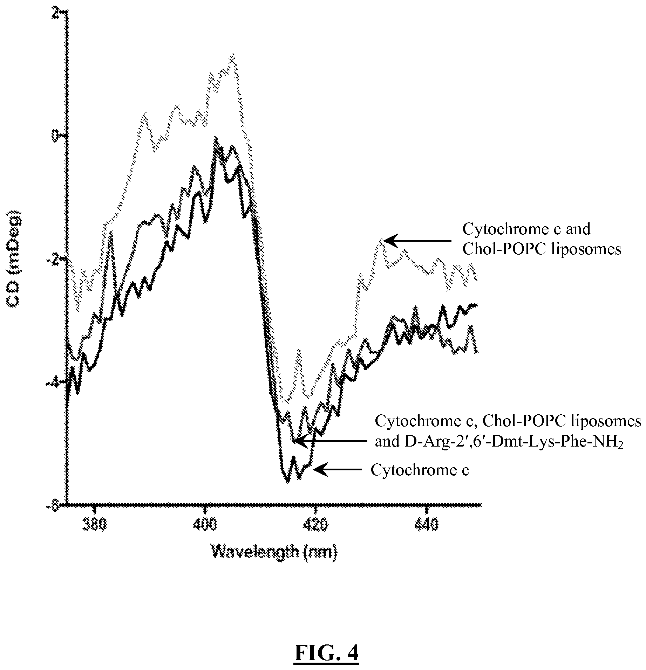

FIG. 4 is a CD spectra showing that addition of the peptide D-Arg-2',6'-Dmt-Lys-Phe-NH₂ (SS-31) inhibits cholesterol-induced structural changes in cytochrome c. The addition of the peptide restored part of the peaks lost due to cholesterol-induced cytochrome c damage. The addition of 40 µM chol-POPC significantly changes the CD spectra, and 100 µM D-Arg-2',6'-Dmt-Lys-Phe-NH₂ (SS-31) nearly restored the spectra to its initial condition.

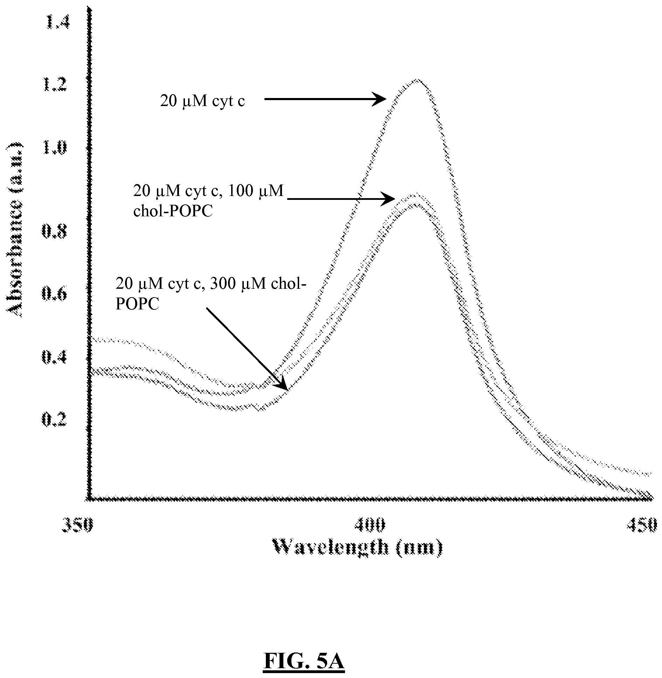

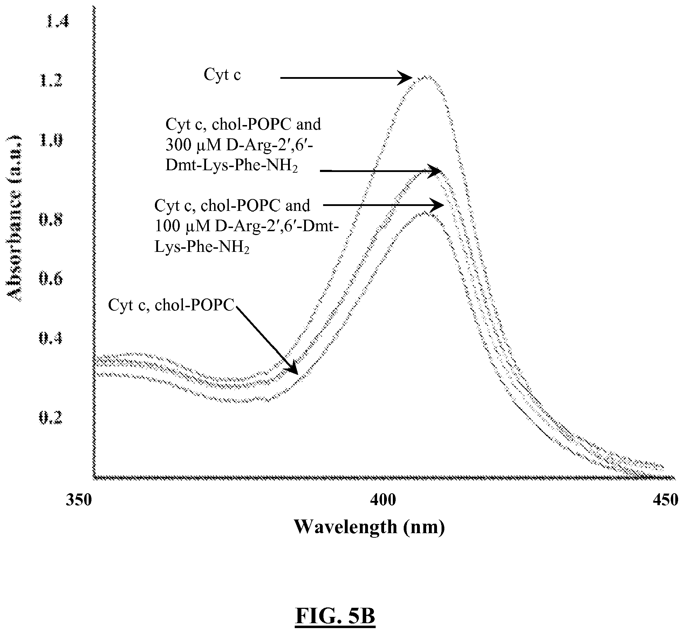

FIG. 5A and 5B are charts showing that the peptide D-Arg-2',6'-Dmt-Lys-Phe-NH₂ (SS-31) reduces cholesterol-induced alterations to the absorbance spectra of cytochrome c. FIG. 5A shows a scan of the absorbance spectra of cytochrome c and cytochrome c incubated with chol-POPC liposomes. These results show significant structural changes to the cytochrome c protein upon exposure to chol-POPC liposomes. FIG. 5B shows that the addition of D-Arg-2',6'-Dmt-Lys-Phe-NH₂ (SS-31) decreases the effect of chol-POPC liposomes on cytochrome c structure.

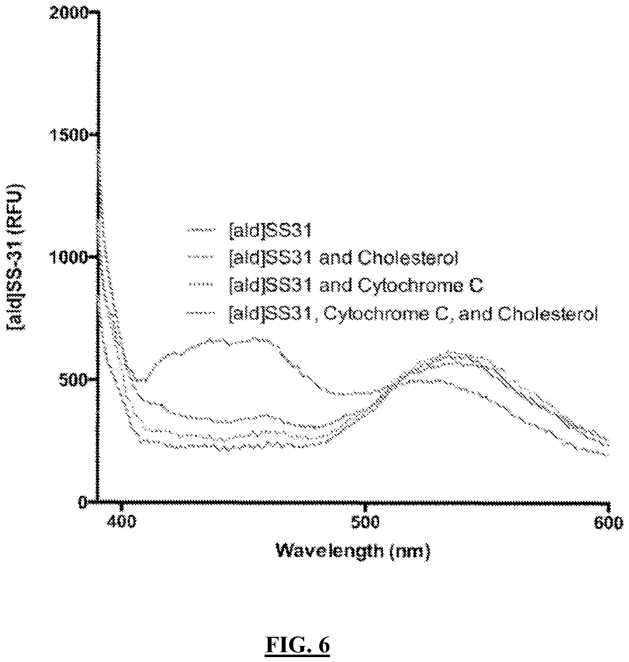

FIG. 6 is a chart showing the emission spectrum of D-Arg-2',6'-Dmt-Lys-Ald-NH₂ ([ald]SS-31). These results show that the peptide blocks the formation of cholesterol-cytochrome c complexes.

### DETAILED DESCRIPTION

The invention is defined in the claims. It is to be appreciated that certain aspects, modes, embodiments, variations and features of the present technology are described below in various levels of detail in order to provide a substantial understanding of the present technology. The definitions of certain terms as used in this specification are provided below. Unless defined otherwise, all technical and scientific terms used herein generally have the same meaning as commonly understood by one of ordinary skill in the art to which this present technology belongs.

In practicing the present disclosure, many conventional techniques of cell biology, molecular biology, protein biochemistry, immunology, and bacteriology are used. These techniques are well-known in the art and are provided in any number of available publications, including Current Protocols in Molecular Biology, Vols. I-III, Ausubel, Ed. (1997); Sambrook et al., Molecular Cloning: A Laboratory Manual, Second Ed. (Cold Spring Harbor Laboratory Press, Cold Spring Harbor, NY, 1989).

As used in this specification and the appended claims, the singular forms "a", "an" and "the" include plural referents unless the content clearly dictates otherwise. For example, reference to "a cell" includes a combination of two or more cells, and the like.

As used herein, the "administration" of an agent, drug, or peptide to a subject includes any route of introducing or delivering to a subject a compound to perform its intended function. Administration can be carried out by any suitable route, including orally, nasally, parenterally (intravenously, intramuscularly, intraperitoneally, or subcutaneously), topically, epicutaneously, rectally, intradermally, transdermally, inhalation, intraarterially, intracerebrally, interosseusly, intrathecally, iontophoretically, ocularly, intravaginaly, *etc.* Administration includes self-administration and the administration by another.

As used herein, the term "amino acid" includes naturally-occurring amino acids and synthetic amino acids, as well as amino acid analogs and amino acid mimetics that function in a manner similar to the naturally-occurring amino acids. Naturally-occurring amino acids are those encoded by the genetic code, as well as those amino acids that are later modified, *e.g.,* hydroxyproline, γ-carboxyglutamate, and O-phosphoserine. Amino acid analogs refers to compounds that have the same basic chemical structure as a naturally-occurring amino acid, *i.e.,* an α-carbon that is bound to a hydrogen, a carboxyl group, an amino group, and an R group, *e.g.,* homoserine, norleucine, methionine sulfoxide, methionine methyl sulfonium. Such analogs have modified R groups (*e.g.,* norleucine) or modified peptide backbones, but retain the same basic chemical structure as a naturally-occurring amino acid. Amino acid mimetics refers to chemical compounds that have a structure that is different from the general chemical structure of an amino acid, but that functions in a manner similar to a naturally-occurring amino acid. Amino acids can be referred to herein by either their commonly known three letter symbols or by the one-letter symbols recommended by the IUPAC-IUB Biochemical Nomenclature Commission.

As used herein, the term "effective amount" refers to a quantity sufficient to achieve a desired therapeutic and/or prophylactic effect. In the context of therapeutic or prophylactic applications, the amount of a composition administered to the subject will depend on the type and severity of the disease and on the characteristics of the individual, such as general health, age, sex, body weight and tolerance to drugs. It will also depend on the degree, severity and type of disease. The skilled artisan will be able to determine appropriate dosages depending on these and other factors. The compositions can also be administered in combination with one or more additional therapeutic compounds.

An "isolated" or "purified" polypeptide or peptide is substantially free of cellular material or other contaminating polypeptides from the cell or tissue source from which the agent is derived, or substantially free from chemical precursors or other chemicals when chemically synthesized. For example, an isolated aromatic-cationic peptide or an isolated cytochrome c protein would be free of materials that would interfere with diagnostic or therapeutic uses of the agent or would interfere with conductance, or electric properties of the peptide. Such interfering materials may include enzymes, hormones and other proteinaceous and nonproteinaceous solutes.

As used herein, the terms "polypeptide", "peptide", and "protein" are used interchangeably herein to mean a polymer comprising two or more amino acids joined to each other by peptide bonds or modified peptide bonds, *i.e.,* peptide isosteres. Polypeptide refers to both short chains, commonly referred to as peptides, glycopeptides or oligomers, and to longer chains, generally referred to as proteins. Polypeptides may contain amino acids other than the 20 gene-encoded amino acids. Polypeptides include amino acid sequences modified either by natural processes, such as post-translational processing, or by chemical modification techniques that are well known in the art.

As used herein, the term "separate" therapeutic use refers to an administration of at least two active ingredients at the same time or at substantially the same time by different routes.

As used herein, the term "sequential" therapeutic use refers to administration of at least two active ingredients at different times, the administration route being identical or different. More particularly, sequential use refers to the whole administration of one of the active ingredients before administration of the other or others commences. It is thus possible to administer one of the active ingredients over several minutes, hours, or days before administering the other active ingredient or ingredients. There is no simultaneous treatment in this case.

As used herein, the term "simultaneous" therapeutic use refers to the administration of at least two active ingredients by the same route and at the same time or at substantially the same time.

As used herein, the terms "subject," "individual," or "patient" can be an individual organism, a vertebrate, a mammal, or a human.

As used herein, a "synergistic therapeutic effect" refers to a greater-than-additive therapeutic effect which is produced by a combination of at least two therapeutic agents, and which exceeds that which would otherwise result from the sole administration of the at least two therapeutic agents. Therefore, lower doses of one or more of the at least two therapeutic agents may be used in treating a disease or disorder, resulting in increased therapeutic efficacy and decreased side-effects.

As used herein, a "therapeutically effective amount" of a compound refers to compound levels in which the physiological effects of a disease or disorder are, at a minimum, ameliorated. A therapeutically effective amount can be given in one or more administrations. The amount of a compound which constitutes a therapeutically effective amount will vary depending on the compound, the disorder and its severity, and the general health, age, sex, body weight and tolerance to drugs of the subject to be treated, but can be determined routinely by one of ordinary skill in the art.

As used herein, the terms "treating" or "treatment" or "alleviation" covers the treatment of a disease or disorder described herein, in a subject, such as a human, and includes: (i) inhibiting a disease or disorder, *i.e.,* arresting its development; (ii) relieving a disease or disorder, *i.e.,* causing regression of the disorder; (iii) slowing progression of the disorder; and/or (iv) inhibiting, relieving, or slowing progression of one or more symptoms of the disease or disorder.

As used herein, "prevention" or "preventing" of a disorder or condition refers to a compound that reduces the occurrence of the disorder or condition in the treated sample relative to an untreated control sample, or delays the onset of one or more symptoms of the disorder or condition relative to the untreated control sample.

It is also to be appreciated that the various modes of treatment or prevention of medical conditions as described are intended to mean "substantial", which includes total but also less than total treatment or prevention, and wherein some biologically or medically relevant result is achieved.

### Aromatic-cationic Peptides

The present technology relates to the use of aromatic-cationic peptides. The peptides can be useful in aspects related to treating or ameliorating symptoms, conditions or diseases characterized by cholesterol-induced mitochondrial dysfunction.

The aromatic-cationic peptides are water-soluble and highly polar. Despite these properties, the peptides can readily penetrate cell membranes. The aromatic-cationic peptides typically include a minimum of three amino acids or a minimum of four amino acids, covalently joined by peptide bonds. The maximum number of amino acids present in the aromatic-cationic peptides is about twenty amino acids covalently joined by peptide bonds. Suitably, the maximum number of amino acids is about twelve, about nine, or about six.

The amino acids of the aromatic-cationic peptides can be any amino acid. As used herein, the term "amino acid" is used to refer to any organic molecule that contains at least one amino group and at least one carboxyl group. Typically, at least one amino group is at the α position relative to a carboxyl group. The amino acids may be naturally occurring. Naturally occurring amino acids include, for example, the twenty most common levorotatory (L) amino acids normally found in mammalian proteins, *i.e.,* alanine (Ala), arginine (Arg), asparagine (Asn), aspartic acid (Asp), cysteine (Cys), glutamine (Gln), glutamic acid (Glu), glycine (Gly), histidine (His), isoleucine (Ile), leucine (Leu), lysine (Lys), methionine (Met), phenylalanine (Phe), proline (Pro), serine (Ser), threonine (Thr), tryptophan, (Trp), tyrosine (Tyr), and valine (Val). Other naturally occurring amino acids include, for example, amino acids that are synthesized in metabolic processes not associated with protein synthesis. For example, the amino acids ornithine and citrulline are synthesized in mammalian metabolism during the production of urea. Another example of a naturally occurring amino acid includes hydroxyproline (Hyp).

The peptides optionally contain one or more non-naturally occurring amino acids. Optimally, the peptide has no amino acids that are naturally occurring. The non-naturally occurring amino acids may be levorotary (L-), dextrorotatory (D-), or mixtures thereof. Non-naturally occurring amino acids are those amino acids that typically are not synthesized in normal metabolic processes in living organisms, and do not naturally occur in proteins. In addition, the non-naturally occurring amino acids suitably are also not recognized by common proteases. The non-naturally occurring amino acid can be present at any position in the peptide. For example, the non-naturally occurring amino acid can be at the N-terminus, the C-terminus, or at any position between the N-terminus and the C-terminus.

The non-natural amino acids may, for example, comprise alkyl, aryl, or alkylaryl groups not found in natural amino acids. Some examples of non-natural alkyl amino acids include α-aminobutyric acid, β-aminobutyric acid, γ-aminobutyric acid, δ-aminovaleric acid, and ε-aminocaproic acid. Some examples of non-natural aryl amino acids include ortho-, meta, and para-aminobenzoic acid. Some examples of non-natural alkylaryl amino acids include ortho-, meta-, and para-aminophenylacetic acid, and γ-phenyl-β-aminobutyric acid. Non-naturally occurring amino acids include derivatives of naturally occurring amino acids. The derivatives of naturally occurring amino acids may, for example, include the addition of one or more chemical groups to the naturally occurring amino acid.

For example, one or more chemical groups can be added to one or more of the 2', 3', 4', 5', or 6' position of the aromatic ring of a phenylalanine or tyrosine residue, or the 4', 5', 6', or 7' position of the benzo ring of a tryptophan residue. The group can be any chemical group that can be added to an aromatic ring. Some examples of such groups include branched or unbranched C₁-C₄ alkyl, such as methyl, ethyl, n-propyl, isopropyl, butyl, isobutyl, or t-butyl, C₁-C₄ alkyloxy (*i.e.,* alkoxy), amino, C₁-C₄ alkylamino and C₁-C₄ dialkylamino (*e.g.,* methylamino, dimethylamino), nitro, hydroxyl, halo (*i.e.,* fluoro, chloro, bromo, or iodo). Some specific examples of non-naturally occurring derivatives of naturally occurring amino acids include norvaline (Nva) and norleucine (Nle).

Another example of a modification of an amino acid in a peptide is the derivatization of a carboxyl group of an aspartic acid or a glutamic acid residue of the peptide. One example of derivatization is amidation with ammonia or with a primary or secondary amine, *e.g.,* methylamine, ethylamine, dimethylamine or diethylamine. Another example of derivatization includes esterification with, for example, methyl or ethyl alcohol. Another such modification includes derivatization of an amino group of a lysine, arginine, or histidine residue. For example, such amino groups can be acylated. Some suitable acyl groups include, for example, a benzoyl group or an alkanoyl group comprising any of the C₁-C₄ alkyl groups mentioned above, such as an acetyl or propionyl group.

The non-naturally occurring amino acids are suitably resistant or insensitive, to common proteases. Examples of non-naturally occurring amino acids that are resistant or insensitive to proteases include the dextrorotatory (D-) form of any of the above-mentioned naturally occurring L-amino acids, as well as L- and/or D- non-naturally occurring amino acids. The D-amino acids do not normally occur in proteins, although they are found in certain peptide antibiotics that are synthesized by means other than the normal ribosomal protein synthetic machinery of the cell. As used herein, the D-amino acids are considered to be non-naturally occurring amino acids.

In order to minimize protease sensitivity, the peptides should have less than five, less than four, less than three, or less than two contiguous L-amino acids recognized by common proteases, irrespective of whether the amino acids are naturally or non-naturally occurring. In one embodiment, the peptide has only D-amino acids, and no L-amino acids. If the peptide contains protease sensitive sequences of amino acids, at least one of the amino acids is preferably a non-naturally-occurring D-amino acid, thereby conferring protease resistance. An example of a protease sensitive sequence includes two or more contiguous basic amino acids that are readily cleaved by common proteases, such as endopeptidases and trypsin. Examples of basic amino acids include arginine, lysine and histidine.

The aromatic-cationic peptides should have a minimum number of net positive charges at physiological pH in comparison to the total number of amino acid residues in the peptide. The minimum number of net positive charges at physiological pH will be referred to below as (pₘ). The total number of amino acid residues in the peptide will be referred to below as (r). The minimum number of net positive charges discussed below are all at physiological pH. The term "physiological pH" as used herein refers to the normal pH in the cells of the tissues and organs of the mammalian body. For instance, the physiological pH of a human is normally approximately 7.4, but normal physiological pH in mammals may be any pH from about 7.0 to about 7.8.

"Net charge" as used herein refers to the balance of the number of positive charges and the number of negative charges carried by the amino acids present in the peptide. In this specification, it is understood that net charges are measured at physiological pH. The naturally occurring amino acids that are positively charged at physiological pH include L-lysine, L-arginine, and L-histidine. The naturally occurring amino acids that are negatively charged at physiological pH include L-aspartic acid and L-glutamic acid.

Typically, a peptide has a positively charged N-terminal amino group and a negatively charged C-terminal carboxyl group. The charges cancel each other out at physiological pH. As an example of calculating net charge, the peptide Tyr-Arg-Phe-Lys-Glu-His-Trp-D-Arg has one negatively charged amino acid (*i.e.,* Glu) and four positively charged amino acids (*i.e.,* two Arg residues, one Lys, and one His). Therefore, the above peptide has a net positive charge of three.

The aromatic-cationic peptides can have a relationship between the minimum number of net positive charges at physiological pH (pₘ) and the total number of amino acid residues (r) wherein 3pₘ is the largest number that is less than or equal to r + 1. In this embodiment, the relationship between the minimum number of net positive charges (pₘ) and the total number of amino acid residues (r) is as follows:

**TABLE 1. Amino acid number and net positive charges (3pₘ ≤ p+1)**

| | | | | | | | | | | | | | | | | | | |
|---|---|---|---|---|---|---|---|---|---|---|---|---|---|---|---|---|---|---|
| **(r)** | 3 | 4 | 5 | 6 | 7 | 8 | 9 | 10 | 11 | 12 | 13 | 14 | 15 | 16 | 17 | 18 | 19 | 20 |
| **(pₘ)** | 1 | 1 | 2 | 2 | 2 | 3 | 3 | 3 | 4 | 4 | 4 | 5 | 5 | 5 | 6 | 6 | 6 | 7 |

The aromatic-cationic peptides can have a relationship between the minimum number of net positive charges (pₘ) and the total number of amino acid residues (r) wherein 2pₘ is the largest number that is less than or equal to r + 1. In this embodiment, the relationship between the minimum number of net positive charges (pₘ) and the total number of amino acid residues (r) is as follows:

**TABLE 2. Amino acid number and net positive charges (2pₘ ≤ p+1)**

| | | | | | | | | | | | | | | | | | | |
|---|---|---|---|---|---|---|---|---|---|---|---|---|---|---|---|---|---|---|
| **(r)** | 3 | 4 | 5 | 6 | 7 | 8 | 9 | 10 | 11 | 12 | 13 | 14 | 15 | 16 | 17 | 18 | 19 | 20 |
| **(pₘ)** | 2 | 2 | 3 | 3 | 4 | 4 | 5 | 5 | 6 | 6 | 7 | 7 | 8 | 8 | 9 | 9 | 10 | 10 |

In one embodiment disclosed herein, the minimum number of net positive charges (pₘ) and the total number of amino acid residues (r) are equal. In another embodiment, the peptides have three or four amino acid residues and a minimum of one net positive charge, suitably, a minimum of two net positive charges and more preferably a minimum of three net positive charges.

It is also important that the aromatic-cationic peptides have a minimum number of aromatic groups in comparison to the total number of net positive charges (pₜ). The minimum number of aromatic groups will be referred to below as (a). Naturally occurring amino acids that have an aromatic group include the amino acids histidine, tryptophan, tyrosine, and phenylalanine. For example, the hexapeptide Lys-Gln-Tyr-D-Arg-Phe-Trp has a net positive charge of two (contributed by the lysine and arginine residues) and three aromatic groups (contributed by tyrosine, phenylalanine and tryptophan residues).

The aromatic-cationic peptides should also have a relationship between the minimum number of aromatic groups (a) and the total number of net positive charges at physiological pH (pₜ) wherein 3a is the largest number that is less than or equal to pₜ + 1, except that when pₜ is 1, a may also be 1. In this embodiment, the relationship between the minimum number of aromatic groups (a) and the total number of net positive charges (pₜ) is as follows:

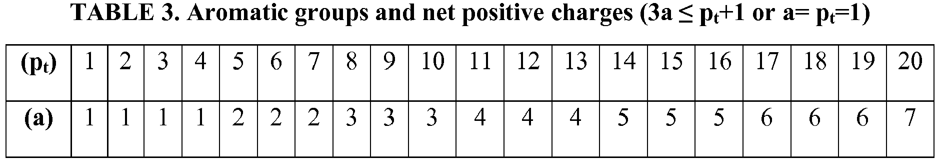

In another embodiment disclosed herein, the aromatic-cationic peptides have a relationship between the minimum number of aromatic groups (a) and the total number of net positive charges (pₜ) wherein 2a is the largest number that is less than or equal to pₜ + 1. In this embodiment, the relationship between the minimum number of aromatic amino acid residues (a) and the total number of net positive charges (pₜ) is as follows:

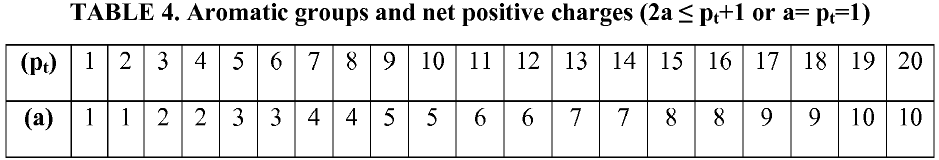

In another embodiment disclosed herein, the number of aromatic groups (a) and the total number of net positive charges (pₜ) are equal.

Carboxyl groups, especially the terminal carboxyl group of a C-terminal amino acid, are suitably amidated with, for example, ammonia to form the C-terminal amide. Alternatively, the terminal carboxyl group of the C-terminal amino acid may be amidated with any primary or secondary amine. The primary or secondary amine may, for example, be an alkyl, especially a branched or unbranched C₁-C₄ alkyl, or an aryl amine. Accordingly, the amino acid at the C-terminus of the peptide may be converted to an amido, N-methylamido, N-ethylamido, N,N-dimethylamido, N,N-diethylamido, N-methyl-N-ethylamido, N-phenylamido or N-phenyl-N-ethylamido group. The free carboxylate groups of the asparagine, glutamine, aspartic acid, and glutamic acid residues not occurring at the C-terminus of the aromatic-cationic peptides may also be amidated wherever they occur within the peptide. The amidation at these internal positions may be with ammonia or any of the primary or secondary amines described above.

In one embodiment disclosed herein, the aromatic-cationic peptide is a tripeptide having two net positive charges and at least one aromatic amino acid. In a particular embodiment, the aromatic-cationic peptide is a tripeptide having two net positive charges and two aromatic amino acids.

In yet another aspect disclosed herein, the present technology provides an aromatic-cationic peptide or a pharmaceutically acceptable salt thereof such as acetate, tartrate, or trifluoroacetate salt. In some embodiments, the peptide comprises
1. at least one net positive charge;
2. a minimum of three amino acids;
3. a maximum of about twenty amino acids;
4. a relationship between the minimum number of net positive charges (pₘ) and the total number of amino acid residues (r) wherein 3pₘ is the largest number that is less than or equal to r + 1; and
5. a relationship between the minimum number of aromatic groups (a) and the total number of net positive charges (pₜ) wherein 2a is the largest number that is less than or equal to pₜ + 1, except that when a is 1, pₜ may also be 1.

In some embodiments, the peptide comprises the amino acid sequence Tyr-D-Arg-Phe-Lys-NH₂ (SS-01), 2',6'-Dmt-D-Arg-Phe-Lys-NH₂ (SS-02), Phe-D-Arg-Phe-Lys-NH₂ (SS-20) or D-Arg-2',6'-Dmt-Lys-Phe-NH₂ (SS-31). In some embodiments also disclosed herein, the peptide comprises one or more of:
D-Arg-Dmt-Lys-Trp-NH₂;
D-Arg-Trp-Lys-Trp-NH₂;
D-Arg-2',6'-Dmt-Lys-Phe-Met-NH₂;
H-D-Arg-Dmt-Lys(*N^{α}*Me)-Phe-NH₂;
H-D-Arg-Dmt-Lys-Phe(*N*Me)-NH₂;
H-D-Arg-Dmt-Lys(*N^{α}*Me)-Phe(*N*Me)-NH₂;
H-D-Arg(*N^{α}*Me)-Dmt(*N*Me)-Lys(*N^{α}*Me)-Phe(*N*Me)-NH₂;
D-Arg-Dmt-Lys-Phe-Lys-Trp-NH₂;
D-Arg-Dmt-Lys-Dmt-Lys-Trp-NH₂;
D-Arg-Dmt-Lys-Phe-Lys-Met-NH₂;
D-Arg-Dmt-Lys-Dmt-Lys-Met-NH₂;
H-D-Arg-Dmt-Lys-Phe-Sar-Gly-Cys-NH₂;
H-D-Arg-Ψ[CH₂-NH]Dmt-Lys-Phe-NH₂;
H-D-Arg-Dmt-Ψ[CH₂-NH]Lys-Phe-NH₂;
H-D-Arg-Dmt-LysΨ[CH₂-NH]Phe-NH₂;
H-D-Arg-Dmt-Ψ[CH₂-NH]Lys-Ψ[CH₂-NH]Phe-NH₂;
Lys-D-Arg-Tyr-NH₂;
Tyr-D-Arg-Phe-Lys-NH₂;
2',6'-Dmt-D-Arg-Phe-Lys-NH₂;
Phe-D-Arg-Phe-Lys-NH₂;
Phe-D-Arg-Dmt-Lys-NH₂;
D-Arg-2'6'Dmt-Lys-Phe-NH₂;
H-Phe-D-Arg-Phe-Lys-Cys-NH₂;
Lys-D-Arg-Tyr-NH₂;
D-Tyr-Trp-Lys-NH₂;
Trp-D-Lys-Tyr-Arg-NH₂;
Tyr-His-D-Gly-Met;
Tyr-D-Arg-Phe-Lys-Glu-NH₂;
Met-Tyr-D-Lys-Phe-Arg;
D-His-Glu-Lys-Tyr-D-Phe-Arg;
Lys-D-Gln-Tyr-Arg-D-Phe-Trp-NH₂;
Phe-D-Arg-Lys-Trp-Tyr-D-Arg-His;
Gly-D-Phe-Lys-Tyr-His-D-Arg-Tyr-NH₂;
Val-D-Lys-His-Tyr-D-Phe-Ser-Tyr-Arg-NH₂;
Trp-Lys-Phe-D-Asp-Arg-Tyr-D-His-Lys;
Lys-Trp-D-Tyr-Arg-Asn-Phe-Tyr-D-His-NH₂;
Thr-Gly-Tyr-Arg-D-His-Phe-Trp-D-His-Lys;
Asp-D-Trp-Lys-Tyr-D-His-Phe-Arg- D-Gly-Lys-NH₂;
D-His-Lys-Tyr-D-Phe-Glu-D-Asp-D-His-D-Lys-Arg-Trp-NH₂;
Ala-D-Phe-D-Arg-Tyr-Lys-D-Trp-His-D-Tyr-Gly-Phe;
Tyr-D-His-Phe- D-Arg-Asp-Lys- D-Arg-His-Trp-D-His-Phe;
Phe-Phe-D-Tyr-Arg-Glu-Asp-D-Lys-Arg-D-Arg-His-Phe-NH₂;
Phe-Tyr-Lys-D-Arg-Trp-His-D-Lys-D-Lys-Glu-Arg-D-Tyr-Thr;
Tyr-Asp-D-Lys-Tyr-Phe- D-Lys- D-Arg-Phe-Pro-D-Tyr-His-Lys;
Glu-Arg-D-Lys-Tyr-D-Val-Phe-D-His-Trp-Arg-D-Gly-Tyr-Arg-D-Met-NH₂;
Arg-D-Leu-D-Tyr-Phe-Lys-Glu- D-Lys-Arg-D-Trp-Lys- D-Phe-Tyr-D-Arg-Gly;
D-Glu-Asp-Lys-D-Arg-D-His-Phe-Phe-D-Val-Tyr-Arg-Tyr-D-Tyr-Arg-His-Phe-NH₂;
Asp-Arg-D-Phe-Cys-Phe-D-Arg-D-Lys-Tyr-Arg-D-Tyr-Trp-D-His-Tyr-D-Phe-Lys-Phe; 
His-Tyr-D-Arg-Trp-Lys-Phe-D-Asp-Ala-Arg-Cys-D-Tyr-His-Phe-D-Lys-Tyr-His-Ser-
NH2;
 
Gly-Ala-Lys-Phe-D-Lys-Glu-Arg-Tyr-His-D-Arg-D-Arg-Asp-Tyr-Trp-D-His-Trp-His-D-
Lys-Asp;
 
Thr-Tyr-Arg-D-Lys-Trp-Tyr-Glu-Asp-D-Lys-D-Arg-His-Phe-D-Tyr-Gly-Val-Ile-D-His-
Arg-Tyr-Lys-NH2;
Dmt-D-Arg-Phe-(atn)Dap-NH₂, where (atn)Dap is β-anthraniloyl-L-α,β-diaminopropionic acid;
Dmt-D-Arg-Ald-Lys-NH₂, where Ald is β-(6'-dimethylamino-2'-naphthoyl)alanine;
Dmt-D-Arg-Phe-Lys-Ald-NH₂, where Ald is β-(6'-dimethylamino-2'-naphthoyl)alanine
Dmt-D-Arg-Phe-(dns)Dap-NH₂ where (dns)Dap is β-dansyl-L-α,β-diaminopropionic acid; D-Arg-Tyr-Lys-Phe-NH₂; and
D-Arg-Tyr-Lys-Phe-NH₂.

In some embodiments, "Dmt" refers to 2',6'-dimethyltyrosine (2',6'-Dmt) or 3',5'-dimethyltyrosine (3'5'Dmt).

In some embodiments disclosed herein, the peptide is defined by formula I: 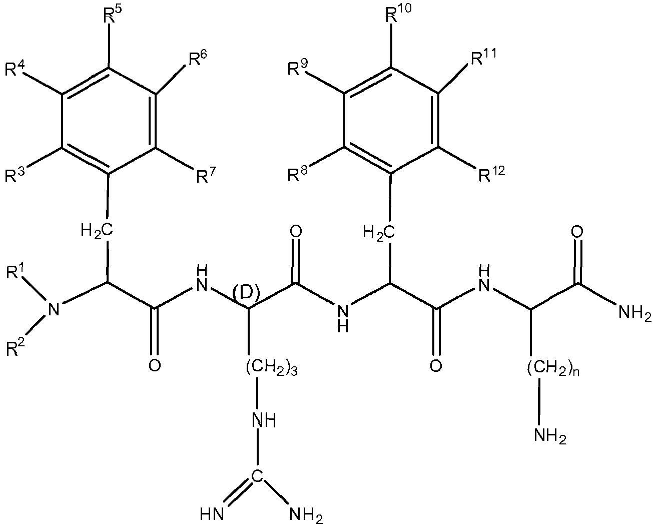
wherein R¹ and R² are each independently selected from
   (i) hydrogen;
   (ii) linear or branched C₁-C₆ alkyl;
   (iii) 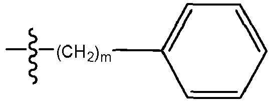 where m = 1-3;
   (iv) 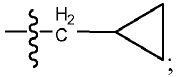
   (v) 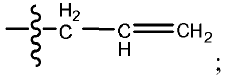
R³, R⁴, R⁵, R⁶, R⁷, R⁸, R⁹, R¹⁰, R¹¹ and R¹² are each independently selected from
   (i) hydrogen;
   (ii) linear or branched C₁-C₆ alkyl;
   (iii) C₁-C₆ alkoxy;
   (iv) amino;
   (v) C₁-C₄ alkylamino;
   (vi) C₁-C₄ dialkylamino;
   (vii) nitro;
   (viii) hydroxyl;
   (ix) halogen, where "halogen" encompasses chloro, fluoro, bromo, and iodo; and
n is an integer from 1 to 5.

In some emobdiments, R¹, R², R³, R⁴, R⁵, R⁶, R⁷, R⁸, R⁹, R¹⁰, R¹¹, and R¹² are all hydrogen; and n is 4. In another embodiment, R¹, R², R³, R⁴, R⁵, R⁶, R⁷, R⁸, R⁹, and R¹¹ are all hydrogen; R⁸ and R¹² are methyl; R¹⁰ is hydroxyl; and n is 4.

In some embodiments disclosed herein, the peptide is defined by formula II: 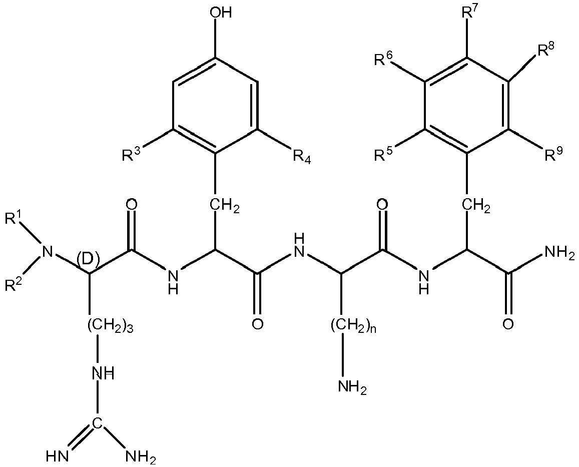
wherein R¹ and R² are each independently selected from
   (i) hydrogen;
   (ii) linear or branched C₁-C₆ alkyl;
   (iii) 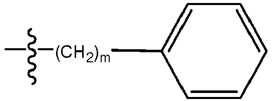 where m = 1-3;
   (iv) 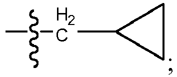
   (v) 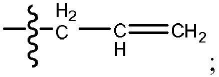
R³ and R⁴ are each independently selected from
   (i) hydrogen;
   (ii) linear or branched C₁-C₆ alkyl;
   (iii) C₁-C₆ alkoxy;
   (iv) amino;
   (v) C₁-C₄ alkylamino;
   (vi) C₁-C₄ dialkylamino;
   (vii) nitro;
   (viii) hydroxyl;
   (ix) halogen, where "halogen" encompasses chloro, fluoro, bromo, and iodo;
R⁵, R⁶, R⁷, R⁸, and R⁹ are each independently selected from
   (i) hydrogen;
   (ii) linear or branched C₁-C₆ alkyl;
   (iii) C₁-C₆ alkoxy;
   (iv) amino;
   (v) C₁-C₄ alkylamino;
   (vi) C₁-C₄ dialkylamino;
   (vii) nitro;
   (viii) hydroxyl;
   (ix) halogen, where "halogen" encompasses chloro, fluoro, bromo, and iodo; and n is an integer from 1 to 5.

In some embodiments disclosed herein, the peptide is defined by the formula: 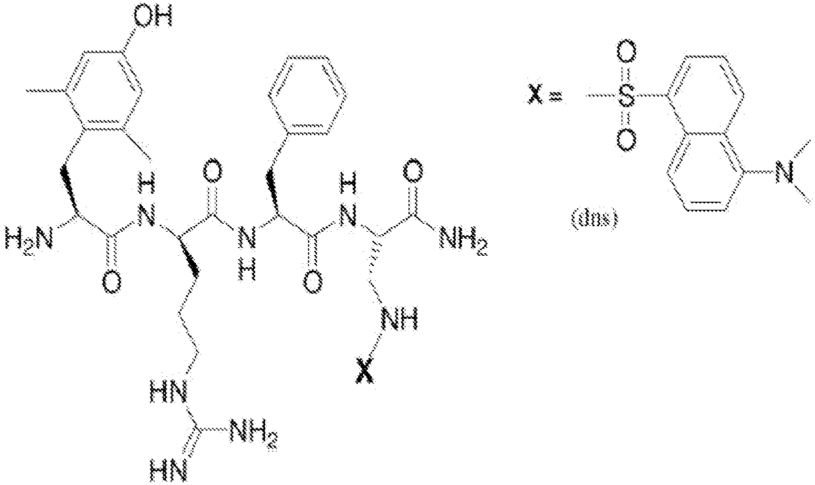 also represented as 2',6'-Dmt-D-Arg-Phe-(dns)Dap-NH₂, where (dns)Dap is β-dansyl-L-α,β-diaminopropionic acid (SS-17).

In some embodiments disclosed herein, the peptide is defined by the formula: 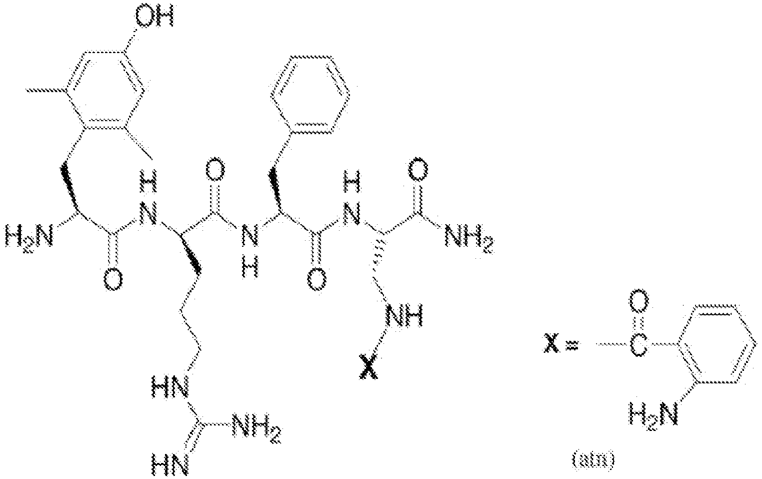 also represented as 2',6'-Dmt-D-Arg-Phe-(atn)Dap-NH₂ where (atn)Dap is β-anthraniloyl-L-α,β-diaminopropionic acid (SS-19). SS-19 is also referred to as [atn]SS-02.

In a particular embodiment, R¹ and R² are hydrogen; R³ and R⁴ are methyl; R⁵, R⁶, R⁷, R⁸, and R⁹ are all hydrogen; and n is 4.

In one embodiment disclosed herein, the aromatic-cationic peptides have a core structural motif of alternating aromatic and cationic amino acids. For example, the peptide may be a tetrapeptide defined by any of formulas III to VI set forth below:
Aromatic - Cationic - Aromatic - Cationic (Formula III)
Cationic - Aromatic - Cationic - Aromatic (Formula IV)
Aromatic - Aromatic - Cationic - Cationic (Formula V)
Cationic - Cationic - Aromatic - Aromatic (Formula VI)
wherein, Aromatic is a residue selected from the group consisting of: Phe (F), Tyr (Y), Trp (W), and Cyclohexylalanine (Cha); and Cationic is a residue selected from the group consisting of: Arg (R), Lys (K), Norleucine (Nle), and 2-amino-heptanoic acid (Ahe).

In some embodiments, the aromatic-cationic peptides described herein comprise all levorotatory (L) amino acids.

In one embodiment, the aromatic-cationic peptide has
1. at least one net positive charge;
2. a minimum of three amino acids;
3. a maximum of about twenty amino acids;
4. a relationship between the minimum number of net positive charges (pₘ) and the total number of amino acid residues (r) wherein 3pₘ is the largest number that is less than or equal to r + 1; and
5. a relationship between the minimum number of aromatic groups (a) and the total number of net positive charges (pₜ) wherein 2a is the largest number that is less than or equal to pₜ + 1, except that when a is 1, pₜ may also be 1.

In another embodiment described herein, the present technology provides a method for reducing the number of mitochondria undergoing a mitochondrial permeability transition (MPT), or preventing mitochondrial permeability transitioning in a removed organ of a mammal or treating or ameliorating symptoms, conditions or diseases characterized by cholesterol-induced mitochondrial dysfunction. The method comprises administering to the removed organ an effective amount of an aromatic-cationic peptide having:
at least one net positive charge;
a minimum of three amino acids;
a maximum of about twenty amino acids;
a relationship between the minimum number of net positive charges (pₘ) and the total number of amino acid residues (r) wherein 3pₘ is the largest number that is less than or equal to r + 1; and
a relationship between the minimum number of aromatic groups (a) and the total number of net positive charges (pₜ) wherein 2a is the largest number that is less than or equal to pₜ + 1, except that when a is 1, pₜ may also be 1.

In yet another embodiment described herein, the present technology provides a method of reducing the number of mitochondria undergoing mitochondrial permeability transition (MPT), or preventing mitochondria permeability transitioning in a mammal in need thereof, or treating or ameliorating symptoms, conditions or diseases characterized by cholesterol-induced mitochondrial dysfunction. The method comprises administering to the mammal an effective amount of an aromatic-cationic peptide having:
at least one net positive charge;
a minimum of three amino acids;
a maximum of about twenty amino acids;
a relationship between the minimum number of net positive charges (pₘ) and the total number of amino acid residues (r) wherein 3 pₘ is the largest number that is less than or equal to r + 1; and
a relationship between the minimum number of aromatic groups (a) and the total number of net positive charges (pₜ) wherein 3a is the largest number that is less than or equal to pₜ + 1, except that when a is 1, pₜ may also be 1.

Aromatic-cationic peptides include, but are not limited to, the following illustrative peptides:
H-Phe-D-Arg Phe-Lys-Cys-NH₂
D-Arg-Dmt-Lys-Trp-NH₂;
D-Arg-Trp-Lys-Trp-NH₂;
D-Arg-Dmt-Lys-Phe-Met-NH₂;
H-D-Arg-Dmt-Lys(*N^{α}*Me)-Phe-NH₂;
H-D-Arg-Dmt-Lys-Phe(*N*Me)-NH₂;
H-D-Arg-Dmt-Lys(*N^{α}*Me)-Phe(*N*Me)-NH₂;
H-D-Arg(*N^{α}*Me)-Dmt(*N*Me)-Lys(*N^{α}*Me)-Phe(*N*Me)-NH₂;
D-Arg-Dmt-Lys-Phe-Lys-Trp-NH₂;
D-Arg-Dmt-Lys-Dmt-Lys-Trp-NH₂;
D-Arg-Dmt-Lys-Phe-Lys-Met-NH₂;
D-Arg-Dmt-Lys-Dmt-Lys-Met-NH₂;
H-D-Arg-Dmt-Lys-Phe-Sar-Gly-Cys-NH₂;
H-D-Arg-Ψ[CH₂-NH]Dmt-Lys-Phe-NH₂;
H-D-Arg-Dmt-Ψ[CH₂-NH]Lys-Phe-NH₂;
H-D-Arg-Dmt-LysΨ[CH₂-NH]Phe-NH₂; and
H-D-Arg-Dmt-Ψ[CH₂-NH]Lys-Ψ[CH₂-NH]Phe-NH₂,
Tyr-D-Arg-Phe-Lys-NH₂,
2',6'-Dmt-D-Arg-Phe-Lys-NH₂,
Phe-D-Arg-Phe-Lys-NH₂,
Phe-D-Arg-Dmt-Lys-NH₂,
D-Arg-2'6'Dmt-Lys-Phe-NH₂,
H-Phe-D-Arg-Phe-Lys-Cys-NH₂,
Lys-D-Arg-Tyr-NH₂,
D-Tyr-Trp-Lys-NH₂,
Trp-D-Lys-Tyr-Arg-NH₂,
Tyr-His-D-Gly-Met,
Tyr-D-Arg-Phe-Lys-Glu-NH₂,
Met-Tyr-D-Lys-Phe-Arg,
D-His-Glu-Lys-Tyr-D-Phe-Arg,
Lys-D-Gln-Tyr-Arg-D-Phe-Trp-NH₂,
Phe-D-Arg-Lys-Trp-Tyr-D-Arg-His,
Gly-D-Phe-Lys-Tyr-His-D-Arg-Tyr-NH₂,
Val-D-Lys-His-Tyr-D-Phe-Ser-Tyr-Arg-NH₂,
Trp-Lys-Phe-D-Asp-Arg-Tyr-D-His-Lys,
Lys-Trp-D-Tyr-Arg-Asn-Phe-Tyr-D-His-NH₂,
Thr-Gly-Tyr-Arg-D-His-Phe-Trp-D-His-Lys,
Asp-D-Trp-Lys-Tyr-D-His-Phe-Arg- D-Gly-Lys-NH₂,
D-His-Lys-Tyr-D-Phe-Glu-D-Asp-D-His-D-Lys-Arg-Trp-NH₂,
Ala-D-Phe-D-Arg-Tyr-Lys-D-Trp-His-D-Tyr-Gly-Phe,
Tyr-D-His-Phe- D-Arg-Asp-Lys- D-Arg-His-Trp-D-His-Phe,
Phe-Phe-D-Tyr-Arg-Glu-Asp-D-Lys-Arg-D-Arg-His-Phe-NH₂,
Phe-Tyr-Lys-D-Arg-Trp-His-D-Lys-D-Lys-Glu-Arg-D-Tyr-Thr,
Tyr-Asp-D-Lys-Tyr-Phe- D-Lys- D-Arg-Phe-Pro-D-Tyr-His-Lys,
Glu-Arg-D-Lys-Tyr-D-Val-Phe-D-His-Trp-Arg-D-Gly-Tyr-Arg-D-Met-NH₂,
Arg-D-Leu-D-Tyr-Phe-Lys-Glu- D-Lys-Arg-D-Trp-Lys- D-Phe-Tyr-D-Arg-Gly,
D-Glu-Asp-Lys-D-Arg-D-His-Phe-Phe-D-Val-Tyr-Arg-Tyr-D-Tyr-Arg-His-Phe-NH₂,
Asp-Arg-D-Phe-Cys-Phe-D-Arg-D-Lys-Tyr-Arg-D-Tyr-Trp-D-His-Tyr-D-Phe-Lys-Phe, 
His-Tyr-D-Arg-Trp-Lys-Phe-D-Asp-Ala-Arg-Cys-D-Tyr-His-Phe-D-Lys-Tyr-His-Ser-
NH2,
 
Gly-Ala-Lys-Phe-D-Lys-Glu-Arg-Tyr-His-D-Arg-D-Arg-Asp-Tyr-Trp-D-His-Trp-His-
D-Lys-Asp, and
 
Thr-Tyr-Arg-D-Lys-Trp-Tyr-Glu-Asp-D-Lys-D-Arg-His-Phe-D-Tyr-Gly-Val-Ile-D-
His-Arg-Tyr-Lys-NH2;
Dmt-D-Arg-Phe-(atn)Dap-NH₂, where (atn)Dap is β-anthraniloyl-L-α,β-diaminopropionic acid;
Dmt-D-Arg-Phe-(dns)Dap-NH₂ where (dns)Dap is β-dansyl-L-α,β-diaminopropionic acid;
Dmt-D-Arg-Ald-Lys-NH₂, where Ald is β-(6'-dimethylamino-2'-naphthoyl)alanine;
Dmt-D-Arg-Phe-Lys-Ald-NH₂, where Ald is β-(6'-dimethylamino-2'-naphthoyl)alanine and D-Arg-Tyr-Lys-Phe-NH₂; and
D-Arg-Tyr-Lys-Phe-NH₂.

In some embodiments described herein, peptides useful in the methods of the present technology are those peptides which have a tyrosine residue or a tyrosine derivative. In some embodiments, derivatives of tyrosine include 2'-methyltyrosine (Mmt); 2',6'-dimethyltyrosine (2'6'Dmt); 3',5'-dimethyltyrosine (3'5'Dmt); N,2',6'-trimethyltyrosine (Tmt); and 2'-hydroxy-6'-methyltryosine (Hmt).

In one embodiment, the peptide has the formula Tyr-D-Arg-Phe-Lys-NH₂ (referred to herein as SS-01). SS-01 has a net positive charge of three, contributed by the amino acids tyrosine, arginine, and lysine and has two aromatic groups contributed by the amino acids phenylalanine and tyrosine. The tyrosine of SS-01 can be a modified derivative of tyrosine such as in 2',6'-dimethyltyrosine to produce the compound having the formula 2',6'-Dmt-D-Arg-Phe-Lys-NH₂ (referred to herein as SS-02).

In a suitable embodiment, the amino acid residue at the N-terminus is arginine. An example of such a peptide is D-Arg-2',6' Dmt-Lys-Phe-NH₂ (referred to herein as SS-31).

In another embodiment, the amino acid at the N-terminus is phenylalanine or its derivative. In some embodiments, derivatives of phenylalanine include 2'-methylphenylalanine (Mmp), 2',6'-dimethylphenylalanine (Dmp), N,2',6'-trimethylphenylalanine (Tmp), and 2'-hydroxy-6'-methylphenylalanine (Hmp). An example of such a peptide is Phe-D-Arg-Phe-Lys-NH₂ (referred to herein as SS-20). In one embodiment, the amino acid sequence of SS-02 is rearranged such that Dmt is not at the N-terminus. An example of such an aromatic-cationic peptide has the formula D-Arg-2',6' Dmt-Lys-Phe-NH₂ (SS-31).

In yet another embodiment, the aromatic-cationic peptide has the formula Phe-D-Arg-Dmt-Lys-NH₂ (referred to herein as SS-30). Alternatively, the N-terminal phenylalanine can be a derivative of phenylalanine such as 2',6'-dimethylphenylalanine (2'6'Dmp). SS-01 containing 2',6'-dimethylphenylalanine at amino acid position one has the formula 2',6'-Dmp-D-Arg-Dmt-Lys-NH₂.

In some embodiments, the aromatic cationic peptide comprises 2',6'-Dmt-D-Arg-Phe-(atn)Dap-NH₂ (SS-19), where (atn)Dap is β-anthraniloyl-L-α,β-diaminopropionic acid, 2',6'-Dmt-D-Arg-Ald-Lys-NH₂ (SS-36), where Ald is β-(6'-dimethylamino-2'-naphthoyl)alanine, 2',6'-Dmt-D-Arg-Phe-Lys-Ald-NH₂ (SS-37), where Ald is β-(6'-dimethylamino-2'-naphthoyl)alanine, D-Arg-Tyr-Lys-Phe-NH₂ (SPI-231), and 2',6'-Dmt-D-Arg-Phe-(dns)Dap-NH₂ where (dns)Dap is β-dansyl-L-α,β-diaminopropionic acid (SS-17).

The peptides mentioned herein and their derivatives can further include functional analogs. A peptide is considered a functional analog if the analog has the same function as the stated peptide. The analog may, for example, be a substitution variant of a peptide, wherein one or more amino acids are substituted by another amino acid. Suitable substitution variants of the peptides include conservative amino acid substitutions. Amino acids may be grouped according to their physicochemical characteristics as follows:
(a) Non-polar amino acids: Ala(A) Ser(S) Thr(T) Pro(P) Gly(G) Cys (C);
(b) Acidic amino acids: Asn(N) Asp(D) Glu(E) Gln(Q);
(c) Basic amino acids: His(H) Arg(R) Lys(K);
(d) Hydrophobic amino acids: Met(M) Leu(L) Ile(I) Val(V); and
(e) Aromatic amino acids: Phe(F) Tyr(Y) Trp(W) His (H).

Substitutions of an amino acid in a peptide by another amino acid in the same group is referred to as a conservative substitution and may preserve the physicochemical characteristics of the original peptide. In contrast, substitutions of an amino acid in a peptide by another amino acid in a different group are generally more likely to alter the characteristics of the original peptide. Non-limiting examples of analogs useful in the practice of the present technology include, but are not limited to, the aromatic-cationic peptides shown in Table 5.

**TABLE 5. Examples of Peptide Analogs**

| **Amino Acid Position 1** | **Amino Acid Position 2** | **Amino Acid Position 3** | **Amino Acid Position 4** | **Amino Acid Position 5** | **Amino Acid Position 6** | **Amino Acid Position 7** | **C-Terminal Modification** |
|---|---|---|---|---|---|---|---|
| D-Arg | Dmt | Lys | Phe | | | | NH₂ |
| D-Arg | Dmt | Phe | Lys | | | | NH₂ |
| D-Arg | Phe | Lys | Dmt | | | | NH₂ |
| D-Arg | Phe | Dmt | Lys | | | | NH₂ |
| D-Arg | Lys | Dmt | Phe | | | | NH₂ |
| D-Arg | Lys | Phe | Dmt | | | | NH₂ |
| D-Arg | Dmt | Lys | Phe | Cys | | | NH₂ |
| D-Arg | Dmt | Lys | Phe | Glu | Cys | Gly | NH₂ |
| D-Arg | Dmt | Lys | Phe | Ser | Cys | | NH₂ |
| D-Arg | Dmt | Lys | Phe | Gly | Cys | | NH₂ |
| Phe | Lys | Dmt | D-Arg | | | | NH₂ |
| Phe | Lys | D-Arg | Dmt | | | | NH₂ |
| Phe | D-Arg | Phe | Lys | | | | NH₂ |
| Phe | D-Arg | Phe | Lys | Cys | | | NH₂ |
| Phe | D-Arg | Phe | Lys | Glu | Cys | Gly | NH₂ |
| Phe | D-Arg | Phe | Lys | Ser | Cys | | NH₂ |
| Phe | D-Arg | Phe | Lys | Gly | Cys | | NH₂ |
| Phe | D-Arg | Dmt | Lys | | | | NH₂ |
| Phe | D-Arg | Dmt | Lys | Cys | | | NH₂ |
| Phe | D-Arg | Dmt | Lys | Glu | Cys | Gly | NH₂ |
| Phe | D-Arg | Dmt | Lys | Ser | Cys | | NH₂ |
| Phe | D-Arg | Dmt | Lys | Gly | Cys | | NH₂ |
| Phe | D-Arg | Lys | Dmt | | | | NH₂ |
| Phe | Dmt | D-Arg | Lys | | | | NH₂ |
| Phe | Dmt | Lys | D-Arg | | | | NH₂ |
| Lys | Phe | D-Arg | Dmt | | | | NH₂ |
| Lys | Phe | Dmt | D-Arg | | | | NH₂ |
| Lys | Dmt | D-Arg | Phe | | | | NH₂ |
| Lys | Dmt | Phe | D-Arg | | | | NH₂ |
| Lys | D-Arg | Phe | Dmt | | | | NH₂ |
| Lys | D-Arg | Dmt | Phe | | | | NH₂ |
| D-Arg | Dmt | D-Arg | Phe | | | | NH₂ |
| D-Arg | Dmt | D-Arg | Dmt | | | | NH₂ |
| D-Arg | Dmt | D-Arg | Tyr | | | | NH₂ |
| D-Arg | Dmt | D-Arg | Trp | | | | NH₂ |
| Trp | D-Arg | Phe | Lys | | | | NH₂ |
| Trp | D-Arg | Tyr | Lys | | | | NH₂ |
| Trp | D-Arg | Trp | Lys | | | | NH₂ |
| Trp | D-Arg | Dmt | Lys | | | | NH₂ |
| D-Arg | Trp | Lys | Phe | | | | NH₂ |
| D-Arg | Trp | Phe | Lys | | | | NH₂ |
| D-Arg | Trp | Lys | Dmt | | | | NH₂ |
| D-Arg | Trp | Dmt | Lys | | | | NH₂ |
| D-Arg | Lys | Trp | Phe | | | | NH₂ |
| D-Arg | Lys | Trp | Dmt | | | | NH₂ |
| Cha | D-Arg | Phe | Lys | | | | NH₂ |
| Ala | D-Arg | Phe | Lys | | | | NH₂ |

| | | | | | | | |
|---|---|---|---|---|---|---|---|
| Cha = cyclohexylalanine | | | | | | | |

Under certain circumstances, it may be advantageous to use a peptide that also has opioid receptor agonist activity. Examples of analogs useful in the practice of the present technology include, but are not limited to, the aromatic-cationic peptides shown in Table 6.

**TABLE 6. Peptide Analogs with Opioid Receptor Agonist Activity**

| **Amino Acid Position 1** | **Amino Acid Position 2** | **Amino Acid Position 3** | **Amino Acid Position 4** | **Amino Acid Position 5 (if present)** | **C-Terminal Modification** |
|---|---|---|---|---|---|
| Tyr | D-Arg | Phe | Lys | | **NH₂** |
| Tyr | D-Arg | Phe | Orn | | NH₂ |
| Tyr | D-Arg | Phe | Dab | | NH₂ |
| Tyr | D-Arg | Phe | Dap | | NH₂ |
| Tyr | D-Arg | Phe | Lys | Cys | NH₂ |
| 2'6'Dmt | D-Arg | Phe | Lys | | NH₂ |
| 2'6'Dmt | D-Arg | Phe | Lys | Cys | NH₂ |
| 2'6'Dmt | D-Arg | Phe | Lys-NH(CH₂)₂-NH-dns | | NH₂ |
| 2'6'Dmt | D-Arg | Phe | Lys-NH(CH₂)₂-NH-atn | | NH₂ |
| 2'6'Dmt | D-Arg | Phe | dnsLys | | NH₂ |
| 2'6'Dmt | D-Cit | Phe | Lys | | NH₂ |
| 2'6'Dmt | D-Cit | Phe | Lys | Cys | NH₂ |
| 2'6'Dmt | D-Cit | Phe | Ahp | | NH₂ |
| 2'6'Dmt | D-Arg | Phe | Orn | | NH₂ |
| 2'6'Dmt | D-Arg | Phe | Dab | | NH₂ |
| 2'6'Dmt | D-Arg | Phe | Dap | | NH₂ |
| 2'6'Dmt | D-Arg | Phe | Ahp(2-aminoheptanoic acid) | | NH₂ |
| Bio-2'6'Dmt | D-Arg | Phe | Lys | | NH₂ |
| 3'5'Dmt | D-Arg | Phe | Lys | | NH₂ |
| 3'5'Dmt | D-Arg | Phe | Orn | | NH₂ |
| 3'5'Dmt | D-Arg | Phe | Dab | | NH₂ |
| 3'5'Dmt | D-Arg | Phe | Dap | | NH₂ |
| Tyr | D-Arg | Tyr | Lys | | NH₂ |
| Tyr | D-Arg | Tyr | Orn | | NH₂ |
| Tyr | D-Arg | Tyr | Dab | | NH₂ |
| Tyr | D-Arg | Tyr | Dap | | NH₂ |
| 2'6'Dmt | D-Arg | Tyr | Lys | | NH₂ |
| 2'6'Dmt | D-Arg | Tyr | Orn | | NH₂ |
| 2'6'Dmt | D-Arg | Tyr | Dab | | NH₂ |
| 2'6'Dmt | D-Arg | Tyr | Dap | | NH₂ |
| 2'6'Dmt | D-Arg | 2'6'Dmt | Lys | | NH₂ |
| 2'6'Dmt | D-Arg | 2'6'Dmt | Orn | | NH₂ |
| 2'6'Dmt | D-Arg | 2'6'Dmt | Dab | | NH₂ |
| 2'6'Dmt | D-Arg | 2'6'Dmt | Dap | | NH₂ |
| 3'5'Dmt | D-Arg | 3'5'Dmt | Arg | | NH₂ |
| 3'5'Dmt | D-Arg | 3'5'Dmt | Lys | | NH₂ |
| 3'5'Dmt | D-Arg | 3'5'Dmt | Orn | | NH₂ |
| 3'5'Dmt | D-Arg | 3'5'Dmt | Dab | | NH₂ |
| 2'6'Dmt | D-Arg | 2'6'Dmt | Lys | Cys | NH₂ |
| Tyr | D-Lys | Phe | Dap | | NH₂ |
| Tyr | D-Lys | Phe | Arg | | NH₂ |
| Tyr | D-Lys | Phe | Arg | Cys | NH₂ |
| Tyr | D-Lys | Phe | Lys | | NH₂ |
| Tyr | D-Lys | Phe | Orn | | NH₂ |
| 2'6'Dmt | D-Lys | Phe | Dab | | NH₂ |
| 2'6'Dmt | D-Lys | Phe | Dap | | NH₂ |
| 2'6'Dmt | D-Lys | Phe | Arg | | NH₂ |
| 2'6'Dmt | D-Lys | Phe | Lys | | NH₂ |
| 3'5'Dmt | D-Lys | Phe | Orn | | NH₂ |
| 3'5'Dmt | D-Lys | Phe | Dab | | NH₂ |
| 3'5'Dmt | D-Lys | Phe | Dap | | NH₂ |
| 3'5'Dmt | D-Lys | Phe | Arg | | NH₂ |
| 3'5'Dmt | D-Lys | Phe | Arg | Cys | NH₂ |
| Tyr | D-Lys | Tyr | Lys | | NH₂ |
| Tyr | D-Lys | Tyr | Orn | | NH₂ |
| Tyr | D-Lys | Tyr | Dab | | NH₂ |
| Tyr | D-Lys | Tyr | Dap | | NH₂ |
| 2'6'Dmt | D-Lys | Tyr | Lys | | NH₂ |
| 2'6'Dmt | D-Lys | Tyr | Orn | | NH₂ |
| 2'6'Dmt | D-Lys | Tyr | Dab | | NH₂ |
| 2'6'Dmt | D-Lys | Tyr | Dap | | NH₂ |
| 2'6'Dmt | D-Lys | 2'6'Dmt | Lys | | NH₂ |
| 2'6'Dmt | D-Lys | 2'6'Dmt | Orn | | NH₂ |
| 2'6'Dmt | D-Lys | 2'6'Dmt | Dab | | NH₂ |
| 2'6'Dmt | D-Lys | 2'6'Dmt | Dap | | NH₂ |
| 2'6'Dmt | D-Arg | Phe | dnsDap | | NH₂ |
| 2'6'Dmt | D-Arg | Phe | atnDap | | NH₂ |
| 3'5'Dmt | D-Lys | 3'5'Dmt | Lys | | NH₂ |
| 3'5'Dmt | D-Lys | 3'5'Dmt | Orn | | NH₂ |
| 3'5'Dmt | D-Lys | 3'5'Dmt | Dab | | NH₂ |
| 3'5'Dmt | D-Lys | 3'5'Dmt | Dap | | NH₂ |
| Tyr | D-Lys | Phe | Arg | | NH₂ |
| Tyr | D-Orn | Phe | Arg | | NH₂ |
| Tyr | D-Dab | Phe | Arg | | NH₂ |
| Tyr | D-Dap | Phe | Arg | | NH₂ |
| 2'6'Dmt | D-Arg | Phe | Arg | | NH₂ |
| 2'6'Dmt | D-Lys | Phe | Arg | | NH₂ |
| 2'6'Dmt | D-Orn | Phe | Arg | | NH₂ |
| 2'6'Dmt | D-Dab | Phe | Arg | | NH₂ |
| 3'5'Dmt | D-Dap | Phe | Arg | | NH₂ |
| 3'5'Dmt | D-Arg | Phe | Arg | | NH₂ |
| 3'5'Dmt | D-Lys | Phe | Arg | | NH₂ |
| 3'5'Dmt | D-Orn | Phe | Arg | | NH₂ |
| Tyr | D-Lys | Tyr | Arg | | NH₂ |
| Tyr | D-Orn | Tyr | Arg | | NH₂ |
| Tyr | D-Dab | Tyr | Arg | | NH₂ |
| Tyr | D-Dap | Tyr | Arg | | NH₂ |
| 2'6'Dmt | D-Arg | 2'6'Dmt | Arg | | NH₂ |
| 2'6'Dmt | D-Lys | 2'6'Dmt | Arg | | NH₂ |
| 2'6'Dmt | D-Orn | 2'6'Dmt | Arg | | NH₂ |
| 2'6'Dmt | D-Dab | 2'6'Dmt | Arg | | NH₂ |
| 3'5'Dmt | D-Dap | 3'5'Dmt | Arg | | NH₂ |
| 3'5'Dmt | D-Arg | 3'5'Dmt | Arg | | NH₂ |
| 3'5'Dmt | D-Lys | 3'5'Dmt | Arg | | NH₂ |
| 3'5'Dmt | D-Orn | 3'5'Dmt | Arg | | NH₂ |
| Mmt | D-Arg | Phe | Lys | | NH₂ |
| Mmt | D-Arg | Phe | Orn | | NH₂ |
| Mmt | D-Arg | Phe | Dab | | NH₂ |
| Mmt | D-Arg | Phe | Dap | | NH₂ |
| Tmt | D-Arg | Phe | Lys | | NH₂ |
| Tmt | D-Arg | Phe | Orn | | NH₂ |
| Tmt | D-Arg | Phe | Dab | | NH₂ |
| Tmt | D-Arg | Phe | Dap | | NH₂ |
| Hmt | D-Arg | Phe | Lys | | NH₂ |
| Hmt | D-Arg | Phe | Orn | | NH₂ |
| Hmt | D-Arg | Phe | Dab | | NH₂ |
| Hmt | D-Arg | Phe | Dap | | NH₂ |
| Mmt | D-Lys | Phe | Lys | | NH₂ |
| Mmt | D-Lys | Phe | Orn | | NH₂ |
| Mmt | D-Lys | Phe | Dab | | NH₂ |
| Mmt | D-Lys | Phe | Dap | | NH₂ |
| Mmt | D-Lys | Phe | Arg | | NH₂ |
| Tmt | D-Lys | Phe | Lys | | NH₂ |
| Tmt | D-Lys | Phe | Orn | | NH₂ |
| Tmt | D-Lys | Phe | Dab | | NH₂ |
| Tmt | D-Lys | Phe | Dap | | NH₂ |
| Tmt | D-Lys | Phe | Arg | | NH₂ |
| Hmt | D-Lys | Phe | Lys | | NH₂ |
| Hmt | D-Lys | Phe | Orn | | NH₂ |
| Hmt | D-Lys | Phe | Dab | | NH₂ |
| Hmt | D-Lys | Phe | Dap | | NH₂ |
| Hmt | D-Lys | Phe | Arg | | NH₂ |
| Mmt | D-Lys | Phe | Arg | | NH₂ |
| Mmt | D-Orn | Phe | Arg | | NH₂ |
| Mmt | D-Dab | Phe | Arg | | NH₂ |
| Mmt | D-Dap | Phe | Arg | | NH₂ |
| Mmt | D-Arg | Phe | Arg | | NH₂ |
| Tmt | D-Lys | Phe | Arg | | NH₂ |
| Tmt | D-Orn | Phe | Arg | | NH₂ |
| Tmt | D-Dab | Phe | Arg | | NH₂ |
| Tmt | D-Dap | Phe | Arg | | NH₂ |
| Tmt | D-Arg | Phe | Arg | | NH₂ |
| Hmt | D-Lys | Phe | Arg | | NH₂ |
| Hmt | D-Orn | Phe | Arg | | NH₂ |
| Hmt | D-Dab | Phe | Arg | | NH₂ |
| Hmt | D-Dap | Phe | Arg | | NH₂ |
| Hmt | D-Arg | Phe | Arg | | NH₂ |

| | | | | | |
|---|---|---|---|---|---|
| Dab = diaminobutyric Dap = diaminopropionic acid Dmt = dimethyltyrosine Mmt = 2'-methyltyrosine Tmt = N, 2',6'-trimethyltyrosine Hmt = 2'-hydroxy,6'-methyltyrosine dnsDap = β-dansyl-L-α,β-diaminopropionic acid atnDap = β-anthraniloyl-L-α,β-diaminopropionic acid Bio = biotin | | | | | |

Additional peptides having opioid receptor agonist activity include 2',6'-Dmt-D-Arg-Ald-Lys-NH₂ (SS-36), where Ald is β-(6'-dimethylamino-2'-naphthoyl)alanine, and 2',6'-Dmt-D-Arg-Phe-Lys-Ald-NH₂ (SS-37), where Ald is β-(6'-dimethylamino-2'-naphthoyl)alanine.

Peptides which have mu-opioid receptor agonist activity are typically those peptides which have a tyrosine residue or a tyrosine derivative at the N-terminus (*i.e.,* the first amino acid position). Suitable derivatives of tyrosine include 2'-methyltyrosine (Mmt); 2',6'-dimethyltyrosine (2'6'-Dmt); 3',5'-dimethyltyrosine (3'5'Dmt); N,2',6'-trimethyltyrosine (Tmt); and 2'-hydroxy-6'-methyltryosine (Hmt).

Peptides that do not have mu-opioid receptor agonist activity generally do not have a tyrosine residue or a derivative of tyrosine at the N-terminus (*i.e.,* amino acid position 1). The amino acid at the N-terminus can be any naturally occurring or non-naturally occurring amino acid other than tyrosine. In one embodiment, the amino acid at the N-terminus is phenylalanine or its derivative. Exemplary derivatives of phenylalanine include 2'-methylphenylalanine (Mmp), 2',6'-dimethylphenylalanine (2',6'-Dmp), N,2',6'-trimethylphenylalanine (Tmp), and 2'-hydroxy-6'-methylphenylalanine (Hmp).

The amino acids of the peptides shown in Tables 5 and 6 may be in either the L- or the D- configuration.

In some embodiments described herein, the aromatic-cationic peptides include at least one arginine and/or at least one lysine residue. In some embodiments, the arginine and/or lysine residue serves as an electron acceptor and participates in proton coupled electron transport. Additionally or alternatively, in some embodiments, the aromatic-cationic peptide comprises a sequence resulting in a "charge-ring-charge-ring" configuration such as exists in SS-31. Additionally or alternatively, in some embodiments the aromatic-cationic peptides include thiol-containing residues, such as cysteine and methionine. In some embodiments, peptides including thiol-containing residues directly donate electrons and reduce cytochrome c. In some embodiments, the aromatic-cationic peptides include a cysteine at the N- and/or at the C-terminus of the peptide.

In some embodiments, peptide multimers are provided. For example in some embodiments, dimers are provided, such as an SS-20 dimer: Phe-D-Arg-Phe-Lys-Phe-D-Arg-Phe-Lys. In some embodiments, the dimer is an SS-31 dimer: D-Arg-2',6'-Dmt-Lys-Phe-D-Arg-2',6'-Dmt-Lys-Phe-NH₂. In some embodiments, the multimers are trimers, tetramers and/or pentamers. In some embodiments, the multimers include combinations of different monomer peptides (*e.g.,* an SS-20 peptide linked to an SS-31 peptide). In some embodiments, these longer analogs are useful as therapeutic molecules.

In some embodiments, the aromatic-cationic peptides described herein comprise all levorotatory (L) amino acids.

### Peptide Synthesis

The peptides may be synthesized by any of the methods well known in the art. Suitable methods for chemically synthesizing the protein include, for example, those described by Stuart and Young in Solid Phase Peptide Synthesis, Second Edition, Pierce Chemical Company (1984), and in Methods Enzymol., 289, Academic Press, Inc, New York (1997).

One way of stabilizing peptides against enzymatic degradation is the replacement of an L-amino acid with a D-amino acid at the peptide bond undergoing cleavage. Aromatic cationic peptide analogs are prepared containing one or more D-amino acid residues in addition to the D-Arg residue already present. Another way to prevent enzymatic degradation is *N*-methylation of the α-amino group at one or more amino acid residues of the peptides. This will prevent peptide bond cleavage by any peptidase. Examples include: H-D-Arg-Dmt-Lys(*N^{α}*Me)-Phe-NH₂; H-D-Arg-Dmt-Lys-Phe(*N*Me)-NH₂; H-D-Arg-Dmt-Lys(*N^{α}*Me)-Phe(*N*Me)-NH₂; and H-D-Arg(*N^{α}*Me)-Dmt(*N*Me)-Lys(*N^{α}*Me)-Phe(*N*Me)-NH₂. *N*^{α}-methylated analogues have lower hydrogen bonding capacity and can be expected to have improved intestinal permeability.

An alternative way to stabilize a peptide amide bond (-CO-NH-) against enzymatic degradation is its replacement with a reduced amide bond (Ψ[CH₂-NH]). This can be achieved with a reductive alkylation reaction between a Boc-amino acid-aldehyde and the amino group of the N-terminal amino acid residue of the growing peptide chain in solid-phase peptide synthesis. The reduced peptide bond is predicted to result in improved cellular permeability because of reduced hydrogen-bonding capacity. Examples include: H-D-Arg-Ψ[CH₂-NH]Dmt-Lys-Phe-NH₂, H-D-Arg-Dmt-Ψ[CH₂-NH]Lys-Phe-NH₂, H-D-Arg-Dmt-LysΨ[CH₂-NH]Phe-NH₂, H-D-Arg-Dmt-Ψ[CH₂-NH]Lys-Ψ[CH₂-NH]Phe-NH₂, *etc*.

### Cholesterol

Cholesterol plays a variety of key physiological roles in cells. Structurally, cholesterol is known to increase membrane order and decrease the fluidity of lipid membranes (Borchman, Cenedella, & Lamba, 1996; Colell, 2003). Functionally, cholesterol is necessary for the production of steroidogenic hormones, bile acids, and vitamin D (Ikonen, 2008; Lucken-Ardjomande, Montessuit, & Martinou, Cell Death Differ. 15(3):484-93 (2008)).

Despite the wide variety of different roles of cholesterol in the cell, mitochondria are typically described as cholesterol deprived. Healthy mitochondria only maintain about 0.5-3% of the entire cell load of cholesterol in order to maintain necessary functions (Van Meer, Voelker, & Feigenson, 2008). However, a plethora of different diseases have been shown to have high mitochondrial cholesterol levels and altered mitochondrial cholesterol homeostasis as a hallmark of pathogenesis, *e.g*., conditions such as Alzheimer's, cancer, atherosclerosis, fibrosis, insulin resistance, ischemia-reperfusion injury, steatohepatitis, Niemman-Pick, pulmonary fibrosis, and metabolic dysfunctions (Colell *et al.,* 2009; Fernandez *et al.,* 2009; Garcia-Ruiz *et al.,* 2009; Mei *et al.,* 2012; Montero *et al.,* 2008; Musso *et al.,* 2013; Ning *et al.,* 2009, Rouslin *et al.,* 1982; Yu *et al.,* 2005; Bosch *et al.,* 2011). All of these diseases are shown to have significant mitochondrial dysfunction and significant lipid peroxidation. Mitochondrial dysfunction includes but is not limited to, reduced respiration, increased reactive oxygen species production, and increased lipid peroxidation.

High mitochondrial cholesterol exerts negative effects on mitochondrial function, although the mechanism by which it does so is yet to be understood. Mitochondria incubated with cholesterol show significant morphological changes (Echegoyen *et al.,* 1993; Yu *et al.,* 2005). Cholesterol exposure results in mitochondrial swelling, and lack of cristae structures (invaginations of the inner mitochondrial membrane (IMM) that are characteristic of mitochondria structure). Cristae are necessary for mitochondrial function to increase the functional surface area of the IMM to produce more ATP (Mannella, 2006). Additionally, incubation of mitochondria with cholesterol show a significant decrease in mitochondrial respiration and the ability to phosphorylate adenosine diphosophate (ADP) in order to become adenosine triphosophate (ATP) (Echegoyen *et al.,* 1993; Rogers *et al.,* 1979; Yu *et al.,* 2005). Also, an increase in mitochondrial cholesterol results in increased production of reactive oxygen species (ROS) such as superoxide (Mei *et al.,* 2012). ROS is known for causing oxidative damage to lipids, proteins, and DNA (Paradies *et al.,* 2000; Paradies *et al.,* 2001; Paradies *et al.,* 2002; Rogers *et al.,* 1979). Consequently, high ROS levels can result in severe cell damage. Cholesterol exposure also decreases the electrical capacity (*i.e.,* membrane potential) across the IMM, which is required to perform necessary functions. (Mei *et al.,* 2012).

### Lipids

Cardiolipin is an important component of the inner mitochondrial membrane, where it constitutes about 20% of the total lipid composition. In mammalian cells, cardiolipin is found almost exclusively in the inner mitochondrial membrane where it is essential for the optimal function of enzymes involved in mitochondrial metabolism.

Cardiolipin is a species of diphosphatidylglycerol lipid comprising two phosphatidylglycerols connected with a glycerol backbone to form a dimeric structure. It has four alkyl groups and potentially carries two negative charges. As there are four distinct alkyl chains in cardiolipin, the molecule has the potential for great complexity. However, in most animal tissues, cardiolipin contains 18-carbon fatty alkyl chains with 2 unsaturated bonds on each of them. It has been proposed that the (18:2)4 acyl chain configuration is an important structural requirement for the high affinity of cardiolipin to inner membrane proteins in mammalian mitochondria. However, studies with isolated enzyme preparations indicate that its importance may vary depending on the protein examined.

Each of the two phosphates in the molecule can capture one proton. Although it has a symmetric structure, ionization of one phosphate happens at different levels of acidity than ionizing both, with pK1 =3 and pK2 > 7.5. Hence, under normal physiological conditions (a pH of approximately 7.0), the molecule may carry only one negative charge. Hydroxyl groups (-OH and -O-) on the phosphate form stable intramolecular hydrogen bonds, forming a bicyclic resonance structure. This structure traps one proton, which is conducive to oxidative phosphorylation.

During the oxidative phosphorylation process catalyzed by Complex IV, large quantities of protons are transferred from one side of the membrane to another side causing a large pH change. Without wishing to be bound by theory, it has been suggested that cardiolipin functions as a proton trap within the mitochondrial membranes, strictly localizing the proton pool and minimizing pH in the mitochondrial intermembrane space. This function is thought to be due to the unique structure of cardiolipin, which, as described above, can trap a proton within the bicyclic structure while carrying a negative charge. Thus, cardiolipin can serve as an electron buffer pool to release or absorb protons to maintain the pH near the mitochondrial membranes.

In addition, cardiolipin has been shown to play a role in apoptosis. An early event in the apoptosis cascade involves cardiolipin. A cardiolipin-specific oxygenase produces cardiolipin-hydroperoxides which causes the lipid to undergo a conformational change. The oxidized cardiolipin then translocates from the inner mitochondrial membrane to the outer mitochondrial membrane where it is thought to form a pore through which cytochrome c is released into the cytosol. Cytochrome c can bind to the IP3 receptor stimulating calcium release, which further promotes the release of cytochrome c. When the cytoplasmic calcium concentration reaches a toxic level, the cell dies. In addition, extra-mitochondrial cytochrome c interacts with apoptotic activating factors, causing the formation of apoptosomal complexes and activation of the proteolytic caspase cascade.

Another consequence is that cytochrome c interacts with cardiolipin on the inner mitochondrial membrane with high affinity and forms a complex with cardiolipin that is non-productive in transporting electrons, but which acts as a cardiolipin-specific oxygenase/peroxidase. Indeed, interaction of cardiolipin with cytochrome c yields a complex whose normal redox potential is about minus (-) 400 mV more negative than that of intact cytochrome c. As a result, the cytochrome c/cardiolipin complex cannot accept electrons from mitochondrial complex III, leading to enhanced production of superoxide whose dismutation yields H₂O₂. The cytochrome c/cardiolipin complex also cannot accept electrons from superoxide. In addition, the high affinity interaction of cardiolipin with cytochrome c results in the activation of cytochrome c into a cardiolipin-specific peroxidase with selective catalytic activity toward peroxidation of polyunsaturated molecular cardiolipin. The peroxidase reaction of the cytochrome c/cardiolipin complex is driven by H₂O₂ as a source of oxidizing equivalents. Ultimately, this activity results in the accumulation of cardiolipin oxidation products, mainly cardiolipin-OOH and their reduction products, cardiolipin-OH. As noted above, it has been shown that oxygenated cardiolipin species play a role in mitochondrial membrane permeabilization and release of pro-apoptotic factors (including cytochrome c itself) into the cytosol. *See e.g.,* Kagan et al., Advanced Drug Delivery Reviews, 61 (2009) 1375-1385; Kagan et al., Mol. Nutr. Food Res. 2009 January; 53(1): 104-114.

Cytochrome c is a globular protein whose major function is to serve as electron carrier from complex III (cytochrome c reductase) to complex IV (cytochrome c oxidase) in the mitochondrial electron transport chain. The prosthetic heme group is attached to the cytochrome c at Cys14 and Cys17, and is additionally bound by two coordinate axial ligands, His18 and Met80. The 6^{th} coordinate binding to Met80 prevents the interaction of the Fe with other ligands such as O₂, H₂O₂, NO, *etc.*

A pool of cytochrome c is distributed in the intermembrane space, with the rest being associated with the IMM *via* both electrostatic and hydrophobic interactions. Cytochrome c is a highly cationic protein (8+ net charge at neutral pH) that can bind loosely to the anionic phospholipid cardiolipin on the IMM *via* electrostatic interaction. And, as noted above, cytochrome c can also bind tightly to cardiolipin *via* hydrophobic interaction. This tight binding of cytochrome c to cardiolipin results from the extension of an acyl chain of cardiolipin out of the lipid membrane and extending into a hydrophobic channel in the interior of cytochrome c (Tuominen *et al.,* 2001; Kalanxhi & Wallace, 2007; Sinabaldi *et al.,* 2010). This leads to the rupture of the Fe-Met80 bond in the cytochrome c heme pocket and results in a change in the heme environment, as shown by the loss of the negative Cotton peak in the Soret band region (Sinabaldi *et al.,* 2008). It also leads to exposure of the heme Fe to H₂O₂ and NO.

Native cytochrome c has poor peroxidase activity because of its 6th coordination. However, upon hydrophobic binding to cardiolipin, cytochrome c undergoes structural changes that breaks the Fe-Met80 coordination and increases the exposure of the heme Fe to H₂O₂, and cytochrome c switches from an electron carrier to a peroxidase, with cardiolipin being the primary substrate (Vladimirov *et al.,* 2006; Basova *et al.,* 2007). As described above, cardiolipin peroxidation results in altered mitochondrial membrane structure, and the release of cytochrome c from the IMM to initiate caspase-mediated cell death.

### Prophylactic and Therapeutic Uses of Aromatic-cationic Peptides

*Determination of the Biological Effect of the Aromatic-Cationic Peptide-Based Therapeutic.* In various embodiments, suitable *in vitro* or *in vivo* assays are performed to determine the effect of a specific aromatic-cationic peptide-based therapeutic and whether its administration is indicated for treatment. In various embodiments, *in vitro* assays can be performed with representative animal models, to determine if a given aromatic-cationic peptide-based therapeutic exerts the desired effect in preventing or treating disease. Compounds for use in therapy can be tested in suitable animal model systems including, but not limited to rats, mice, chicken, pigs, cows, monkeys, rabbits, and the like, prior to testing in human subjects. Similarly, for *in vivo* testing, any of the animal model systems known in the art can be used prior to administration to human subjects.

*Prophylactic Methods.* In one aspect, the present technology provides a method for preventing a cholesterol-induced disease or condition in a subject by administering to the subject an aromatic-cationic peptide that prevents the initiation or progression of the disease or condition. In prophylactic applications, pharmaceutical compositions or medicaments of aromatic-cationic peptides are administered to a subject susceptible to, or otherwise at risk of a disease or condition in an amount sufficient to eliminate or reduce the risk, or delay the outset of the disease, including biochemical, histologic and/or behavioral symptoms of the disease, its complications and intermediate pathological phenotypes presenting during development of the disease. Administration of a prophylactic aromatic-cationic can occur prior to the manifestation of symptoms characteristic of the aberrancy, such that a disease or disorder is prevented or, alternatively, delayed in its progression. The appropriate compound can be determined based on screening assays described above.

*Therapeutic Methods.* Another aspect of the technology includes methods of treating disease in a subject for therapeutic purposes. In therapeutic applications, compositions or medicaments are administered to a subject suspected of, or already suffering from such a disease in an amount sufficient to cure, reduce the severity or at least partially arrest, the symptoms of the disease, including its complications and intermediate pathological phenotypes in development of the disease.

In one aspect, the disclosure provides a method of reducing the number of mitochondria undergoing cholesterol-induced mitochondrial permeability transition (MPT), or preventing cholesterol-induced mitochondrial permeability transitioning in a mammal in need thereof, the method comprising administering to the mammal an effective amount of one or more aromatic-cationic peptides described herein.

In yet another aspect, the disclosure provides a method for reducing cholesterol-induced oxidative damage in a subject in need thereof, the method comprising administering to the mammal an effective amount of one or more aromatic-cationic peptides described herein. In some embodiments, the aromatic-cationic peptides of the present technology are useful in methods for reducing cholesterol-induced ROS production in a subject in need thereof.

In one aspect, the disclosure provides a method for treating, preventing or ameliorating cholesterol-induced mitochondrial dysfunction in a subject in need thereof, the method comprising administering to the mammal an effective amount of one or more aromatic-cationic peptides described herein.

In some embodiments, the aromatic-cationic peptides of the present technology are useful in methods for inhibiting cholesterol-induced loss of membrane potential in a subject in need thereof.

In some embodiments, the aromatic-cationic peptides of the present technology are useful in methods for inhibiting cholesterol-induced decrease in cellular respiration in a subject in need thereof.

In some embodiments, the aromatic-cationic peptides of the present technology are useful in methods for inhibiting cholesterol-induced decrease in ATP synthesis rate in a subject in need thereof.

In some embodiments, the aromatic-cationic peptides of the present technology are useful in methods for treating, ameliorating or reversing cholesterol-induced mitochondrial damage, *e.g*., loss of cristae formation, in a subject in need thereof.

In some embodiments, the aromatic-cationic peptides of the present technology are useful in methods for promoting glutathione (GSH) transport into the mitochondria in a subject in need thereof. In certain embodiments, the aromatic-cationic peptides of the present technology are useful in methods for treating, ameliorating or reversing cholesterol-induced blockage of GSH transport into the mitochondria in a subject in need thereof.

In some embodiments, the aromatic-cationic peptides of the present technology are useful in methods for inhibiting cholesterol-induced cytochrome c peroxidase activity in a subject in need thereof.

In some embodiments, the aromatic-cationic peptides of the present technology are useful in methods for inhibiting or reversing cholesterol-induced damage to cytochrome c in a subject in need thereof.

In some embodiments, the aromatic-cationic peptides of the present technology are useful in methods for inhibiting cholesterol-induced cardiolipin peroxidation in a subject in need thereof.

In some embodiments, the aromatic-cationic peptides of the present technology are useful in methods for regulating mitochondrial cholesterol levels. In certain embodiments, the aromatic-cationic peptides of the present technology are useful in methods for preventing or treating any cellular damage caused by mitochondrial cholesterol overload.

Thus, in some embodiments, aromatic-cationic peptides as disclosed herein (such as D-Arg-2',6'-Dmt-Lys-Phe-NH₂, Phe-D-Arg-Phe-Lys-NH₂, or a pharmaceutically acceptable salt thereof, such as acetate, tartrate or trifluoroacetate salt) are administered to a subject in need thereof. Without wishing to be bound by theory, it is thought that the peptides contact (*e.g*., target) cytochrome c, cardiolipin or both, hinder the cholesterol-induced cardiolipin- cytochrome c interaction, inhibit the cholesterol-induced oxygenase/peroxidase activity of the cardiolipin/cytochrome c complex, inhibit cholesterol-induced cardiolipin-hydroperoxide formation, inhibit cholesterol-induced translocation of cardiolipin to the outer membrane and/or inhibit the cholesterol-induced release of cytochrome c from the IMM. Additionally or alternatively, in some embodiments, the aromatic-cationic peptides disclosed herein include one or more of the following characteristics or functions: (1) are cell permeable and target the inner mitochondrial membrane; (2) selectively bind to cardiolipin *via* electrostatic interactions which facilitates the interaction of the peptide with cytochrome c; (3) interact with cytochrome c that is free and either loosely-bound or tightly-bound to cardiolipin; (4) protect the hydrophobic heme pocket of cytochrome c and/or inhibit cardiolipin from disrupting the Fe-Met80 bond; (5) promote π-π* interactions with the heme porphyrin; (6) inhibit cytochrome c peroxidase activity; (7) promote kinetics of cytochrome c reduction; (8) prevent inhibition of cytochrome c reduction caused by cardiolipin; (9) promote electron flux in the mitochondrial electron transport chain and ATP synthesis. In some embodiments, the ability of the peptide to promote electron transport is not correlated with the ability of the peptide to inhibit cholesterol-induced peroxidase activity of the cytochrome c/cardiolipin complex. Thus, in some embodiments, the administered peptides inhibit, delay or reduce the cholesterol-induced interaction between cardiolipin and cytochrome c. Additionally or alternatively, in some embodiments, the administered peptides inhibit, delay or reduce the cholesterol-induced formation of cytochrome c/cardiolipin complexes. Additionally or alternatively, in some embodiments, the administered peptides inhibit, delay or reduce the cholesterol-induced oxygenase/peroxidase activity of the cytochrome c/cardiolipin complexes. Additionally or alternatively, in some embodiments, the administered peptides inhibit, delay or reduce the cholesterol-induced increase in cardiolipin peroxidation. Additionally or alternatively, in some embodiments, the administered peptides inhibit, delay or reduce cholesterol-induced increase in apoptosis.

Additionally or alternatively, in some embodiments, the method relates to increasing cytochrome c reduction in a sample containing cytochrome c and cholesterol, comprising contacting the sample with an effective amount of an aromatic-cationic peptide or a salt thereof, such as acetate, tartrate or trifluoroacetate salt. Additionally or alternatively, in some embodiments, the method relates to enhancing electron diffusion through cytochrome c in a sample containing cytochrome c and cholesterol, comprising contacting the sample with an effective amount of an aromatic-cationic peptide. Additionally or alternatively, in some embodiments, the method relates to enhancing electron capacity in cytochrome c in a sample containing cytochrome c and cholesterol, comprising contacting the sample with an effective amount of an aromatic-cationic peptide. Additionally or alternatively, in some embodiments, the method relates to inducing a novel π-π interaction around cytochrome c in a sample containing cytochrome c and cholesterol, comprising contacting the sample with an effective amount of an aromatic-cationic peptide. In some embodiments, the aromatic-cationic peptide comprises D-Arg-2',6'-Dmt-Lys-Phe-NH₂. Additionally or alternatively, in some embodiments, the aromatic-cationic peptide comprises Phe-D-Arg-Phe-Lys-NH₂. In some embodiments, the method includes contacting the sample with an aromatic cationic peptide (e.g., D-Arg-2',6'-Dmt-Lys-Phe-NH₂ or Phe-D-Arg-Phe-Lys-NH₂) and cardiolipin. In some embodiments, the method includes contacting the sample with cardiolipin.

The aromatic-cationic peptides described herein are useful to prevent or treat disease. Specifically, the disclosure provides for both prophylactic and therapeutic methods of treating a subject at risk of (or susceptible to) a disease characterized by cholesterol-induced mitochondrial dysfunction by administering the aromatic-cationic peptides described herein. Accordingly, the present methods provide for the prevention and/or treatment of a disease characterized by cholesterol-induced mitochondrial dysfunction in a subject by administering an effective amount of an aromatic-cationic peptide to a subject in need thereof.

*Oxidative Damage.* The peptides disclosed herein are useful in reducing oxidative damage in a mammal in need thereof. Mammals in need of reducing oxidative damage are those mammals suffering from a disease, condition or treatment associated with oxidative damage. Typically, the oxidative damage is caused by free radicals, such as reactive oxygen species (ROS) and/or reactive nitrogen species (RNS). Examples of ROS and RNS include hydroxyl radical, superoxide anion radical, nitric oxide, hydrogen, hypochlorous acid (HOCl) and peroxynitrite anion. Oxidative damage is considered to be "reduced" if the amount of oxidative damage in a subject, a removed organ, or a cell is decreased after administration of an effective amount of the aromatic cationic peptides described above. Typically, the oxidative damage is considered to be reduced if the oxidative damage is decreased by at least about 10%, at least about 25%, at least about 50%, at least about 75%, or at least about 90%, compared to a control subject not treated with the peptide.

In some embodiments, a subject to be treated can be a mammal with a disease or condition associated with oxidative damage. The oxidative damage can occur in any cell, tissue or organ of the mammal. In humans, oxidative stress is involved in many diseases such as atherosclerosis, Alzheimer's disease *etc.*

In some embodiments, a mammal may be undergoing a treatment associated with oxidative damage. For example, the mammal may be undergoing reperfusion. Reperfusion refers to the restoration of blood flow to any organ or tissue in which the flow of blood is decreased or blocked. The restoration of blood flow during reperfusion leads to respiratory burst and formation of free radicals.

In one embodiment, the mammal may have decreased or blocked blood flow due to hypoxia or ischemia. The loss or severe reduction in blood supply during hypoxia or ischemia may, for example, be due to thromboembolic stroke, coronary atherosclerosis, or peripheral vascular disease. Numerous organs and tissues are subject to ischemia or hypoxia. Examples of such organs include brain, heart, kidney, intestine and prostate. The tissue affected is typically muscle, such as cardiac, skeletal, or smooth muscle For instance, cardiac muscle ischemia or hypoxia is commonly caused by atherosclerotic or thrombotic blockages which lead to the reduction or loss of oxygen delivery to the cardiac tissues by the cardiac arterial and capillary blood supply. Such cardiac ischemia or hypoxia may cause pain and necrosis of the affected cardiac muscle, and ultimately may lead to cardiac failure.

The methods can also be used in reducing oxidative damage associated with any neurodegenerative disease or condition. The neurodegenerative disease can affect any cell, tissue or organ of the central and peripheral nervous system. Examples of such cells, tissues and organs include, the brain, spinal cord, neurons, ganglia, Schwann cells, astrocytes, oligodendrocytes and microglia. The neurodegenerative condition can be an acute condition, such as a stroke or a traumatic brain or spinal cord injury. In another embodiment, the neurodegenerative disease or condition can be a chronic neurodegenerative condition. In a chronic neurodegenerative condition, the free radicals can, for example, cause damage to a protein. An example of such a protein is amyloid β-protein. Examples of chronic neurodegenerative diseases associated with damage by free radicals include Parkinson's disease, Alzheimer's disease, Huntington's disease and Amyotrophic Lateral Sclerosis (also known as Lou Gehrig's disease).

*Mitochondrial Permeability Transitioning.* The peptides disclosed herein are useful in treating any disease or condition that is associated with mitochondria permeability transitioning (MPT). Such diseases and conditions include, but are not limited to, ischemia and/or reperfusion of a tissue or organ, hypoxia and any of a number of neurodegenerative diseases. Mammals in need of inhibiting or preventing of MPT are those mammals suffering from these diseases or conditions.

*Apoptosis.* The peptides disclosed herein are useful in treating diseases or conditions that are associated with apoptosis. Exemplary diseases or conditions include, but are not limited to, cancers such as colorectal, glioma, hepatic, neuroblastoma, leukemias and lymphomata, and prostate; autoimmune diseases such as myasthenia gravis, systemic lupus erythematosus, inflammatory diseases, bronchial asthma, inflammatory intestinal disease, pulmonary inflammation; viral infections such as adenovirus and baculovirus and HIV-AIDS; neurodegenerative diseases such as Alzheimer's disease, amyotrophic lateral sclerosis, Parkinson's disease, retinitis pigmentosa and epilepsy; hematologic diseases such as aplastic anemia, myelodysplastic syndrome, T CD4+ lymphocytopenia, and G6PD deficiency; tissue damage such as caused by myocardial infarction, cerebrovascular accident, ischemic renal damage and polycystic kidney. Thus, in some embodiments, aromatic-cationic peptides as disclosed herein (such as D-Arg-2',6'-Dmt-Lys-Phe-NH₂, Phe-D-Arg-Phe-Lys-NH₂, or a pharmaceutically acceptable salt thereof, such as acetate, tartrate or trifluoroacetate salt) are administered to a subject (*e.g.,* a mammal such as a human) in need thereof. As noted above, it is thought that the peptides contact (*e.g.,* target) cytochrome c, cardiolipin or both, hinder the cholesterol-induced cardiolipin- cytochrome c interaction, inhibit cholesterol-induced cardiolipin-hydroperoxide formation, inhibit the cholesterol-induced translocation of cardiolipin to the outer membrane, and/or inhibit the cholesterol-induced oxygenase/peroxidase activity. Thus, in some embodiments, the administered peptides inhibit, delay or reduce the cholesterol-induced interaction between cardiolipin and cytochrome c. Additionally or alternatively, in some embodiments, the administered peptides inhibit, delay or reduce the cholesterol-induced formation of cytochrome c/cardiolipin complexes. Additionally or alternatively, in some embodiments, the administered peptides inhibit, delay or reduce the cholesterol-induced oxygenase/peroxidase activity of the cytochrome c/cardiolipin complexes. Additionally or alternatively, in some embodiments, the administered peptides inhibit, delay or reduce cholesterol-induced apoptosis.

*Cardiolipin oxidation.* The peptides disclosed herein are useful to prevent, inhibit or diminish cardiolipin oxidation. Cardiolipin is a unique phospholipid that is almost exclusively found on the inner mitochondrial membrane and at contact sites connecting the outer membrane and inner membrane. Cardiolipin domains are required for proper cristae formation and they are involved in the organization of respiratory complexes into higher-order supercomplexes to facilitate electron transfer. Cardiolipin is also required to keep cytochrome c in close proximity to the respiratory complexes for efficient electron transfer. The positively-charged cytochrome c interacts with the highly anionic cardiolipin *via* electrostatic interactions.

This cardiolipin-cytochrome c interaction is weakened by cardiolipin peroxidation, and the loss of free cytochrome c into the cytosol initiates caspase-dependent apoptosis (Shidoji et al., 1999, Biochem Biophys Res Commun 264, 343-347). A remodeling of lipid membranes occurs during apoptosis, and phosphatidylserine moves from the inner leaflet of the plasma membrane to the outer leaflet. In a similar fashion, cardiolipin and its metabolites can relocate from the mitochondria to other intracellular organelles and to the cell surface (Sorice et al., 2004, Cell Death Differ 11, 1133-1145).

Aromatic-cationic peptides disclosed herein (such as but not limited to 2',6'-Dmt-D-Arg-Phe-Lys-NH₂ (SS-02), Phe-D-Arg-Phe-Lys-NH₂ (SS-20) and D-Arg-2',6'-Dmt-Lys-Phe-NH₂ (SS-31), or pharmaceutically acceptable salts thereof, such as acetate, tartrate and trifluoroacetate salt), are useful to prevent cardiolipin peroxidation. Cardiolipin peroxidation is primarily mediated by the peroxidase activity of cytochrome c. About 15-20% of mitochondrial cytochrome c is tightly bound in a complex with cardiolipin *via* hydrophobic interaction whereby one or more acyl chains of cardiolipin is inserted into the hydrophobic channel of cytochrome c. The insertion of the acyl chain into cytochrome c disrupts the coordinate bond between Met80 and the heme Fe and exposes the sixth coordinate of heme Fe to H₂O₂, thus converting this enzyme into a peroxidase that can selectively catalyze the oxidation of cardiolipin.

The aromatic-cationic peptides disclosed herein, (such as but not limited to 2',6'-Dmt-D-Arg-Phe-Lys-NH₂ (SS-02), Phe-D-Arg-Phe-Lys-NH₂ (SS-20) and D-Arg-2',6'-Dmt-Lys-Phe-NH2 (SS-31), or pharmaceutically acceptable salts thereof, such as acetate, tartrate and trifluoroacetate salt, are cell-permeable and can selectively target and concentrate in the inner mitochondrial membrane. The aromatic-cationic peptides selectively target anionic phospholipids such as cardiolipin, phosphatidylserine, phosphatidic acid, and phosphatidylglycerol, with cardiolipin having the highest affinity with some peptides. In some embodiments, the aromatic-cationic peptides interact with cytochrome c and the interaction is enhanced in the presence of cardiolipin. In some embodiments, the peptides can penetrate within the cytochrome c protein in close proximity of the heme, and this penetration is enhanced in the presence of cardiolipin.

Without wishing to be bound by theory, by penetrating into the heme environment of cytochrome c, it is likely that the aromatic-cationic peptides interfere with the structural interaction between cardiolipin and cytochrome c, and prevent the rupture of the Met80-Fe coordinate and prevent cytochrome c from becoming a peroxidase. These aromatic-cationic peptides also increase π-π* interaction in the heme region and promote cytochrome c reduction.

The aromatic-cationic peptides disclosed herein can also enhance mitochondrial respiration, increase oxidative phosphorylation capacity, and reduce mitochondrial reactive oxygen species. As a result, they can protect mitochondrial function, reduce cardiolipin peroxidation, and/or inhibit apoptosis.

### Niemann-Pick Disease

The peptides disclosed herein are useful in treating diseases or conditions characterized by cholesterol-induced mitochondrial dysfunction, such as Alzheimer's, cancer, atherosclerosis, fibrosis, insulin resistance, ischemia-reperfusion injury, Niemann-Pick, non-alcoholic steatohepatitis (NASH), pulmonary fibrosis, and metabolic dysfunctions.

Niemann-Pick Disease refers to a group of inherited severe metabolic disorders (including Niemann-Pick Type C (NPC)) that allow sphingomyelin to accumulate in lysosomes. NPC patients are not able to metabolize cholesterol and other lipids properly within the cell. Consequently, excessive amounts of cholesterol accumulate within the mitochondrial membranes of liver, spleen and neuronal cells. Additionally, the mitochondrial membrane potential and ATP levels are markedly decreased in NPC mouse brains and neurons. It has been shown that reducing the level of cholesterol within mitochondrial membranes using methyl-β-cyclodextrin can restore the activity of ATP synthase. Yu et al., J. Biol. Chem. 280:11731-11739 (2005). Further, NPC neurons show an impaired neurite outgrowth, which can be rescued by exogenous ATP. These results suggest that mitochondrial dysfunctions and subsequent ATP deficiency, may be responsible for neuronal impairment in NPC disease. Niemann- Pick Disease symptoms include difficulty moving limbs (dystonia), enlargement of the spleen and liver (hepatosplenomegaly), dementia, slurred, irregular speech (dysarthria), discoordinated swallowing (dysphagia), sudden loss of muscle tone which may lead to falls (cataplexy), tremors, difficulties moving the eyes up and down (vertical supranuclear gaze palsy), ataxia, reduced appetite, abdominal distension, thrombocytopenia, jaundice, pain, enlarged bone marrow cavities, thinned cortical bone, distortion of the hip bone, seizures, cherry red spot in the eye, and sleep related disorders.

Cardiolipin peroxidation and increased oxidative stress, may be an important process in the pathology of Niemann-Pick Disease. Accordingly, the aromatic-cationic peptides of the present disclosure (such as but not limited to 2',6'-Dmt-D-Arg-Phe-Lys-NH₂ (SS-02), Phe-D-Arg-Phe-Lys-NH₂ (SS-20) and D-Arg-2',6'-Dmt-Lys-Phe-NH₂ (SS-31), or pharmaceutically acceptable salts thereof, such as acetate, tartrate and trifluoroacetate salt) can be used to treat Niemann-Pick Disease by preventing cardiolipin oxidation, oxidative stress, and cellular apoptosis, in a subject.

### Aromatic-cationic Peptides in Electron Transfer

Mitochondrial ATP synthesis is driven by electron flow through the electron transport chain (ETC) of the IMM. Electron flow through the chain can be described as a series of oxidation/reduction processes. Electrons pass from electron donors (NADH or QH2), through a series of electron acceptors (Complexes I-IV), and ultimately to the terminal electron acceptor, molecular oxygen. Cytochrome c, which is loosely associated with the IMM, transfers electrons between Complexes III and IV.

Rapid shunting of electrons through the ETC is important for preventing short-circuiting that would lead to electron escape and generation of free radical intermediates. The rate of electron transfer (ET) between an electron donor and electron acceptor decreases exponentially with the distance between them, and superexchange ET is limited to 20Å. Long-range ET can be achieved in a multi-step electron hopping process, where the overall distance between donor and acceptor is split into a series of shorter, and therefore faster, ET steps. In the ETC, efficient ET over long distances is assisted by cofactors that are strategically localized along the IMM, including FMN, FeS clusters, and hemes. Aromatic amino acids such as Phe, Tyr and Trp can also facilitate electron transfer to heme through overlapping *π* clouds. Amino acids with suitable oxidation potential (Tyr, Trp, Cys, Met) can act as stepping stones by serving as intermediate electron carriers. In addition, the hydroxyl group of Tyr can lose a proton when it conveys an electron, and the presence of a basic group nearby, such as Lys, can result in proton-coupled ET which is even more efficient.

Overexpression of catalase targeted to mitochondria (mCAT) has been shown to improve aging (e.g., reduce the symptoms) and prolong lifespan in mice. Such examples identify "druggable" chemical compounds that can reduce mitochondrial oxidative stress and protect mitochondrial function. As mitochondria are the major source of intracellular reactive oxygen species (ROS), the antioxidant must be delivered to mitochondria in order to limit oxidative damage to mitochondrial DNA, proteins of the electron transport chain (ETC), and the mitochondrial lipid membranes. In some embodiments, aromatic-cationic peptides selectively target and concentrate in the IMM. Some of these peptides contain redox-active amino acids that can undergo one-electron oxidation and behave as mitochondria-targeted antioxidants. The peptides disclosed herein, such as the peptide D-Arg-2'6'-Dmt-Tyr-Lys-Phe-NH₂ reduces mitochondrial ROS and protect mitochondrial function in cellular and animal studies. Recent studies show that this peptide can confer protection against mitochondrial oxidative stress comparable to that observed with mitochondrial catalase overexpression. Although radical scavenging is the most commonly used approach to reduce oxidative stress, there are other potential mechanisms that can be used, including facilitation of electron transfer to reduce electron leak and improved mitochondrial reduction potential.

Abundant circumstantial evidence indicates that oxidative stress contributes to many consequences of normal aging and several major diseases, including cardiovascular diseases, diabetes, neurodegenerative diseases, and cancer. Oxidative stress is generally defined as an imbalance of prooxidants and antioxidants. However, despite a wealth of scientific evidence to support increased oxidative tissue damage, large-scale clinical studies with antioxidants have not demonstrated significant health benefits in these diseases. One of the reasons may be due to the inability of the available antioxidants to reach the site of prooxidant production.

The mitochondrial electron transport chain (ETC) is the primary intracellular producer of ROS, and mitochondria themselves are most vulnerable to oxidative stress. Protecting mitochondrial function would therefore be a prerequisite to preventing cell death caused by mitochondrial oxidative stress. The benefits of overexpressing catalase targeted to mitochondria (mCAT), but not peroxisomes (pCAT), provided proof-of-concept that mitochondria-targeted antioxidants would be necessary to overcome the detrimental effects of aging. However, adequate delivery of chemical antioxidants to the IMM remains a challenge.

One peptide analog, D-Arg-2',6'-Dmt-Tyr-Lys-Phe-NH₂, possesses intrinsic antioxidant ability because the modified tyrosine residue is redox-active and can undergo one-electron oxidation. This peptide can neutralize H₂O₂, hydroxyl radical, and peroxynitrite, and inhibit lipid peroxidation. The peptide has demonstrated remarkable efficacy in animal models of ischemia-reperfusion injury, neurodegenerative diseases, and metabolic syndrome.

The design of the mitochondria-targeted peptides incorporates and enhances one or more of the following modes of action: (i) scavenging excess ROS, (ii) reducing ROS production by facilitating electron transfer, or (iii) increasing mitochondrial reductive capacity. The advantage of peptide molecules is that it is possible to incorporate natural or unnatural amino acids that can serve as redox centers, facilitate electron transfer, or increase sulfydryl groups while retaining the aromatic-cationic motif required for mitochondria targeting.

### Modes of Administration and Effective Dosages

Any method known to those in the art for contacting a cell, organ or tissue with an aromatic-cationic peptide may be employed. Suitable methods include *in vitro, ex vivo, or in vivo* methods. *In vitro* methods typically include cultured samples. For example, a cell can be placed in a reservoir (*e.g.,* tissue culture plate), and incubated with an aromatic-cationic peptide under appropriate conditions suitable for obtaining the desired result. Suitable incubation conditions can be readily determined by those skilled in the art.

*Ex vivo* methods typically include cells, organs or tissues removed from a mammal, such as a human. The cells, organs or tissues can, for example, be incubated with the aromatic-cationic peptide under appropriate conditions. The contacted cells, organs or tissues are typically returned to the donor, placed in a recipient, or stored for future use. Thus, the aromatic-cationic peptide is generally in a pharmaceutically acceptable carrier.

*In vivo* methods typically include the administration of an aromatic-cationic peptide, such as those described above, to a mammal, suitably a human. When used *in vivo* for therapy, the aromatic-cationic peptides are administered to the subject in effective amounts (*i.e.,* amounts that have desired therapeutic effect). The dose and dosage regimen will depend upon the degree of the injury in the subject, the characteristics of the particular aromatic-cationic peptide used, *e.g.,* its therapeutic index, the subject, and the subject's history. The effective amount may be determined during pre-clinical trials and clinical trials by methods familiar to physicians and clinicians.

An effective amount of an aromatic-cationic peptide useful in the methods may be administered to a mammal in need thereof by any of a number of well-known methods for administering pharmaceutical compounds. The aromatic-cationic peptide may be administered systemically or locally.

The aromatic-cationic peptide may be formulated as a pharmaceutically acceptable salt. The term "pharmaceutically acceptable salt" means a salt prepared from a base or an acid which is acceptable for administration to a patient, such as a mammal (*e.g.,* salts having acceptable mammalian safety for a given dosage regime). However, it is understood that the salts are not required to be pharmaceutically acceptable salts, such as salts of intermediate compounds that are not intended for administration to a patient. Pharmaceutically acceptable salts can be derived from pharmaceutically acceptable inorganic or organic bases and from pharmaceutically acceptable inorganic or organic acids. In addition, when a peptide contains both a basic moiety, such as an amine, pyridine or imidazole, and an acidic moiety such as a carboxylic acid or tetrazole, zwitterions may be formed and are included within the term "salt" as used herein. Salts derived from pharmaceutically acceptable inorganic bases include ammonium, calcium, copper, ferric, ferrous, lithium, magnesium, manganic, manganous, potassium, sodium, and zinc salts, and the like. Salts derived from pharmaceutically acceptable organic bases include salts of primary, secondary and tertiary amines, including substituted amines, cyclic amines, naturally-occurring amines and the like, such as arginine, betaine, caffeine, choline, N,N'-dibenzylethylenediamine, diethylamine, 2-diethylaminoethanol, 2-dimethylaminoethanol, ethanolamine, ethylenediamine, N-ethylmorpholine, N-ethylpiperidine, glucamine, glucosamine, histidine, hydrabamine, isopropylamine, lysine, methylglucamine, morpholine, piperazine, piperadine, polyamine resins, procaine, purines, theobromine, triethylamine, trimethylamine, tripropylamine, tromethamine and the like. Salts derived from pharmaceutically acceptable inorganic acids include salts of boric, carbonic, hydrohalic (hydrobromic, hydrochloric, hydrofluoric or hydroiodic), nitric, phosphoric, sulfamic and sulfuric acids. Salts derived from pharmaceutically acceptable organic acids include salts of aliphatic hydroxyl acids (*e.g.,* citric, gluconic, glycolic, lactic, lactobionic, malic, and tartaric acids), aliphatic monocarboxylic acids (*e.g.,* acetic, butyric, formic, propionic and trifluoroacetic acids), amino acids (*e.g.,* aspartic and glutamic acids), aromatic carboxylic acids (*e.g.,* benzoic, p-chlorobenzoic, diphenylacetic, gentisic, hippuric, and triphenylacetic acids), aromatic hydroxyl acids (*e.g.,* o-hydroxybenzoic, p-hydroxybenzoic, 1-hydroxynaphthalene-2-carboxylic and 3-hydroxynaphthalene-2-carboxylic acids), ascorbic, dicarboxylic acids (*e.g.,* fumaric, maleic, oxalic and succinic acids), glucoronic, mandelic, mucic, nicotinic, orotic, pamoic, pantothenic, sulfonic acids (*e.g.,* benzenesulfonic, camphosulfonic, edisylic, ethanesulfonic, isethionic, methanesulfonic, naphthalenesulfonic, naphthalene-1,5-disulfonic, naphthalene-2,6-disulfonic and p-toluenesulfonic acids), xinafoic acid, and the like. In some embodiments, the salt is an acetate salt. In some embodiments, the salt is a tartrate salt. Additionally or alternatively, in other embodiments, the salt is a trifluoroacetate salt.

The aromatic-cationic peptides described herein can be incorporated into pharmaceutical compositions for administration, singly or in combination, to a subject for the treatment or prevention of a disorder described herein. Such compositions typically include the active agent and a pharmaceutically acceptable carrier. As used herein the term "pharmaceutically acceptable carrier" includes saline, solvents, dispersion media, coatings, antibacterial and antifungal agents, isotonic and absorption delaying agents, and the like, compatible with pharmaceutical administration. Supplementary active compounds can also be incorporated into the compositions.

Pharmaceutical compositions are typically formulated to be compatible with its intended route of administration. Examples of routes of administration include parenteral (*e.g.,* intravenous, intradermal, intraperitoneal or subcutaneous), oral, inhalation, transdermal (topical), intraocular, iontophoretic, and transmucosal administration. Solutions or suspensions used for parenteral, intradermal, or subcutaneous application can include the following components: a sterile diluent such as water for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulfite; chelating agents such as ethylenediaminetetraacetic acid; buffers such as acetates, citrates or phosphates and agents for the adjustment of tonicity such as sodium chloride or dextrose. pH can be adjusted with acids or bases, such as hydrochloric acid or sodium hydroxide. The parenteral preparation can be enclosed in ampoules, disposable syringes or multiple dose vials made of glass or plastic. For convenience of the patient or treating physician, the dosing formulation can be provided in a kit containing all necessary equipment (*e.g.,* vials of drug, vials of diluent, syringes and needles) for a treatment course (*e.g.,* 7 days of treatment).

Pharmaceutical compositions suitable for injectable use can include sterile aqueous solutions (where water soluble) or dispersions and sterile powders for the extemporaneous preparation of sterile injectable solutions or dispersion. For intravenous administration, suitable carriers include physiological saline, bacteriostatic water, Cremophor EL™ (BASF, Parsippany, N.J.) or phosphate buffered saline (PBS). In all cases, a composition for parenteral administration must be sterile and should be fluid to the extent that easy syringability exists. It should be stable under the conditions of manufacture and storage and must be preserved against the contaminating action of microorganisms such as bacteria and fungi.

In one embodiment, the aromatic-cationic peptides of the present technology are administered intravenously. For example, an aromatic-cationic peptide may be administered *via* rapid intravenous bolus injection. In some embodiments, the aromatic-cationic peptide is administered as a constant-rate intravenous infusion.

The aromatic-cationic peptide may also be administered orally, topically, intranasally, intramuscularly, subcutaneously, or transdermally. In one embodiment, transdermal administration is by iontophoresis, in which the charged composition is delivered across the skin by an electric current.

Other routes of administration include intracerebroventricularly or intrathecally. Intracerebroventricularly refers to administration into the ventricular system of the brain. Intrathecally refers to administration into the space under the arachnoid membrane of the spinal cord. Thus, in some embodiments, intracerebroventricular or intrathecal administration is used for those diseases and conditions which affect the organs or tissues of the central nervous system.

The aromatic-cationic peptide may also be administered to mammals by sustained release, as is known in the art. Sustained release administration is a method of drug delivery to achieve a certain level of the drug over a particular period of time. The level is typically measured by serum or plasma concentration. A description of methods for delivering a compound by controlled release can be found in international PCT Application No. WO 02/083106.

Any formulation known in the art of pharmacy is suitable for administration of the aromatic-cationic peptide. For oral administration, liquid or solid formulations may be used. Examples of formulations include tablets, gelatin capsules, pills, troches, elixirs, suspensions, syrups, wafers, chewing gum and the like. The aromatic-cationic peptides can be mixed with a suitable pharmaceutical carrier (vehicle) or excipient as understood by practitioners in the art. Examples of carriers and excipients include starch, milk, sugar, certain types of clay, gelatin, lactic acid, stearic acid or salts thereof, including magnesium or calcium stearate, talc, vegetable fats or oils, gums and glycols.

For systemic, intracerebroventricular, intrathecal, topical, intranasal, subcutaneous, or transdermal administration, formulations of the aromatic-cationic peptides may utilize conventional diluents, carriers, or excipients *etc.,* such as those known in the art to deliver the aromatic-cationic peptides. For example, the formulations may comprise one or more of the following: a stabilizer, a surfactant, preferably a nonionic surfactant, and optionally a salt and/or a buffering agent. The aromatic-cationic peptide may be delivered in the form of an aqueous solution, or in a lyophilized form.

The stabilizer may comprise, for example, an amino acid, such as for instance, glycine; an oligosaccharide, such as, sucrose, tetralose, lactose; or a dextran. Alternatively, the stabilizer may comprise a sugar alcohol, such as, mannitol. In some embodiments, the stabilizer or combination of stabilizers constitutes from about 0.1% to about 10% weight for weight of the formulated composition.

In some embodiments, the surfactant is a nonionic surfactant, such as a polysorbate. Examples of suitable surfactants include Tween 20, Tween 80; a polyethylene glycol or a polyoxyethylene polyoxypropylene glycol, such as Pluronic F-68 at from about 0.001% (w/v) to about 10% (w/v).

The salt or buffering agent may be any salt or buffering agent, such as for example, sodium chloride, or sodium/potassium phosphate, respectively. In some embodiments, the buffering agent maintains the pH of the pharmaceutical composition in the range of about 5.5 to about 7.5. The salt and/or buffering agent is also useful to maintain the osmolality at a level suitable for administration to a human or an animal. In some embodiments, the salt or buffering agent is present at a roughly isotonic concentration of about 150 mM to about 300 mM.

Formulations of aromatic-cationic peptides may additionally contain one or more conventional additives. Examples of such additives include a solubilizer such as, for example, glycerol; an antioxidant such as for example, benzalkonium chloride (a mixture of quaternary ammonium compounds, known as "quats"), benzyl alcohol, chloretone or chlorobutanol; an anesthetic agent such as for example a morphine derivative; and an isotonic agent *etc.,* such as described herein. As a further precaution against oxidation or other spoilage, the pharmaceutical compositions may be stored under nitrogen gas in vials sealed with impermeable stoppers.

The mammal treated in accordance with the present technology may be any mammal, including, for example, farm animals, such as sheep, pigs, cows, and horses; pet animals, such as dogs and cats; and laboratory animals, such as rats, mice and rabbits. In one embodiment, the mammal is a human.

In some embodiments, aromatic-cationic peptides are administered to a mammal in an amount effective in reducing the number of mitochondria undergoing, or preventing, MPT. The effective amount is determined during pre-clinical trials and clinical trials by methods familiar to physicians and clinicians.

The aromatic-cationic peptide may be administered systemically or locally. In one embodiment, the aromatic-cationic peptides are administered intravenously. For example, aromatic-cationic peptides may be administered *via* rapid intravenous bolus injection. In one embodiment, the aromatic-cationic peptide is administered as a constant-rate intravenous infusion.

The aromatic-cationic peptide can be injected directly into a coronary artery during, for example, angioplasty or coronary bypass surgery, or applied onto coronary stents.

The aromatic-cationic peptide compositions can include a carrier, which can be a solvent or dispersion medium containing, for example, water, ethanol, polyol (for example, glycerol, propylene glycol, and liquid polyethylene glycol, and the like), and suitable mixtures thereof. The proper fluidity can be maintained, for example, by the use of a coating such as lecithin, by the maintenance of the required particle size in the case of dispersion and by the use of surfactants. Prevention of the action of microorganisms can be achieved by various antibacterial and antifungal agents, for example, parabens, chlorobutanol, phenol, ascorbic acid, thiomerasol, and the like. Glutathione and other antioxidants can be included to prevent oxidation. In some embodiments, isotonic agents, *e.g*., sugars, polyalcohols such as mannitol, sorbitol, or sodium chloride are included in the composition. Prolonged absorption of the injectable compositions can be brought about by including in the composition an agent which delays absorption, for example, aluminum monostearate or gelatin.

Sterile injectable solutions can be prepared by incorporating the active compound in the required amount in an appropriate solvent with one or a combination of ingredients enumerated above, as required, followed by filtered sterilization. Generally, dispersions are prepared by incorporating the active compound into a sterile vehicle, which contains a basic dispersion medium and the required other ingredients from those enumerated above. In the case of sterile powders for the preparation of sterile injectable solutions, typical methods of preparation include vacuum drying and freeze drying, which can yield a powder of the active ingredient plus any additional desired ingredient from a previously sterile-filtered solution thereof.

Oral compositions generally include an inert diluent or an edible carrier. For the purpose of oral therapeutic administration, the active compound can be incorporated with excipients and used in the form of tablets, troches, or capsules, *e.g*., gelatin capsules. Oral compositions can also be prepared using a fluid carrier for use as a mouthwash. Pharmaceutically compatible binding agents, and/or adjuvant materials can be included as part of the composition. The tablets, pills, capsules, troches and the like can contain any of the following ingredients, or compounds of a similar nature: a binder such as microcrystalline cellulose, gum tragacanth or gelatin; an excipient such as starch or lactose, a disintegrating agent such as alginic acid, Primogel, or corn starch; a lubricant such as magnesium stearate or Sterotes; a glidant such as colloidal silicon dioxide; a sweetening agent such as sucrose or saccharin; or a flavoring agent such as peppermint, methyl salicylate, or orange flavoring.

For administration by inhalation, the compounds can be delivered in the form of an aerosol spray from a pressurized container or dispenser which contains a suitable propellant, *e.g.,* a gas such as carbon dioxide, or a nebulizer. Such methods include those described in U.S. Pat. No. 6,468,798.

Systemic administration of a therapeutic compound as described herein can also be by transmucosal or transdermal means. For transmucosal or transdermal administration, penetrants appropriate to the barrier to be permeated are used in the formulation. Such penetrants are generally known in the art, and include, for example, for transmucosal administration, detergents, bile salts, and fusidic acid derivatives. Transmucosal administration can be accomplished through the use of nasal sprays. For transdermal administration, the active compounds are formulated into ointments, salves, gels, or creams as generally known in the art. In one embodiment, transdermal administration may be performed by iontophoresis.

A therapeutic protein or peptide can be formulated in a carrier system. The carrier can be a colloidal system. The colloidal system can be a liposome, a phospholipid bilayer vehicle. In one embodiment, the therapeutic peptide is encapsulated in a liposome while maintaining peptide integrity. As one skilled in the art would appreciate, there are a variety of methods to prepare liposomes. (*See* Lichtenberg et al., Methods Biochem. Anal., 33:337-462 (1988); Anselem et al., Liposome Technology, CRC Press (1993)). Liposomal formulations can delay clearance and increase cellular uptake *(See* Reddy, Ann. Pharmacother., 34(7-8):915-923 (2000)). An active agent can also be loaded into a particle prepared from pharmaceutically acceptable ingredients including, but not limited to, soluble, insoluble, permeable, impermeable, biodegradable or gastroretentive polymers or liposomes. Such particles include, but are not limited to, nanoparticles, biodegradable nanoparticles, microparticles, biodegradable microparticles, nanospheres, biodegradable nanospheres, microspheres, biodegradable microspheres, capsules, emulsions, liposomes, micelles and viral vector systems.

The carrier can also be a polymer, *e.g.,* a biodegradable, biocompatible polymer matrix. In one embodiment, the therapeutic peptide can be embedded in the polymer matrix, while maintaining protein integrity. The polymer may be natural, such as polypeptides, proteins or polysaccharides, or synthetic, such as poly α-hydroxy acids. Examples include carriers made of, *e.g.,* collagen, fibronectin, elastin, cellulose acetate, cellulose nitrate, polysaccharide, fibrin, gelatin, and combinations thereof. In one embodiment, the polymer is poly-lactic acid (PLA) or copoly lactic/glycolic acid (PGLA). The polymeric matrices can be prepared and isolated in a variety of forms and sizes, including microspheres and nanospheres. Polymer formulations can lead to prolonged duration of therapeutic effect. (*See* Reddy, Ann. Pharmacother., 34(7-8):915-923 (2000)). A polymer formulation for human growth hormone (hGH) has been used in clinical trials. *(See* Kozarich and Rich, Chemical Biology, 2:548-552 (1998)).

Examples of polymer microsphere sustained release formulations are described in PCT publication WO 99/15154 (Tracy et al.)*,* U.S. Pat. Nos. 5,674,534 and 5,716,644 (both to Zale et al.)*,* PCT publication WO 96/40073 (Zale et al.)*,* and PCT publication WO 00/38651 (Shah et al.)*.* U.S. Pat. Nos. 5,674,534 and 5,716,644 and PCT publication WO 96/40073 describe a polymeric matrix containing particles of erythropoietin that are stabilized against aggregation with a salt.

In some embodiments, the therapeutic compounds are prepared with carriers that will protect the therapeutic compounds against rapid elimination from the body, such as a controlled release formulation, including implants and microencapsulated delivery systems. Biodegradable, biocompatible polymers can be used, such as ethylene vinyl acetate, polyanhydrides, polyglycolic acid, collagen, polyorthoesters, and polylacetic acid. Such formulations can be prepared using known techniques. The materials can also be obtained commercially, *e.g*., from Alza Corporation and Nova Pharmaceuticals, Inc. Liposomal suspensions (including liposomes targeted to specific cells with monoclonal antibodies to cell-specific antigens) can also be used as pharmaceutically acceptable carriers. These can be prepared according to methods known to those skilled in the art, for example, as described in U.S. Pat. No. 4,522,811.

The therapeutic compounds can also be formulated to enhance intracellular delivery. For example, liposomal delivery systems are known in the art, see, *e.g.,* Chonn and Cullis, "Recent Advances in Liposome Drug Delivery Systems," Current Opinion in Biotechnology 6:698-708 (1995); Weiner, "Liposomes for Protein Delivery: Selecting Manufacture and Development Processes," Immunomethods, 4(3):201-9 (1994); and Gregoriadis, "Engineering Liposomes for Drug Delivery: Progress and Problems," Trends Biotechnol., 13(12):527-37 (1995). Mizguchi et al., Cancer Lett., 100:63-69 (1996), describes the use of fusogenic liposomes to deliver a protein to cells both *in vivo* and *in vitro.*

Dosage, toxicity and therapeutic efficacy of the therapeutic agents can be determined by standard pharmaceutical procedures in cell cultures or experimental animals, *e.g.,* for determining the LD50 (the dose lethal to 50% of the population) and the ED50 (the dose therapeutically effective in 50% of the population). The dose ratio between toxic and therapeutic effects is the therapeutic index and it can be expressed as the ratio LD50/ED50. Compounds which exhibit high therapeutic indices are preferred. While compounds that exhibit toxic side effects may be used, care should be taken to design a delivery system that targets such compounds to the site of affected tissue in order to minimize potential damage to uninfected cells and, thereby, reduce side effects.

The data obtained from the cell culture assays and animal studies can be used in formulating a range of dosage for use in humans. The dosage of such compounds lies preferably within a range of circulating concentrations that include the ED50 with little or no toxicity. The dosage may vary within this range depending upon the dosage form employed and the route of administration utilized. For any compound used in the methods, the therapeutically effective dose can be estimated initially from cell culture assays. A dose can be formulated in animal models to achieve a circulating plasma concentration range that includes the IC50 (*i.e.,* the concentration of the test compound which achieves a half-maximal inhibition of symptoms) as determined in cell culture. Such information can be used to more accurately determine useful doses in humans. Levels in plasma may be measured, for example, by high performance liquid chromatography.

Typically, an effective amount of the aromatic-cationic peptides, sufficient for achieving a therapeutic or prophylactic effect, range from about 0.000001 mg per kilogram body weight per day to about 10,000 mg per kilogram body weight per day. Suitably, the dosage ranges are from about 0.0001 mg per kilogram body weight per day to about 100 mg per kilogram body weight per day. For example dosages can be 1 mg/kg body weight or 10 mg/kg body weight every day, every two days or every three days or within the range of 1-10 mg/kg every week, every two weeks or every three weeks. In one embodiment, a single dosage of peptide ranges from 0.1-10,000 micrograms per kg body weight. In one embodiment, aromatic-cationic peptide concentrations in a carrier range from 0.2 to 2000 micrograms per delivered milliliter. An exemplary treatment regime entails administration once per day or once a week. In therapeutic applications, a relatively high dosage at relatively short intervals is sometimes required until progression of the disease is reduced or terminated, and preferably until the subject shows partial or complete amelioration of symptoms of disease. Thereafter, the patient can be administered a prophylactic regime.

In some embodiments, a therapeutically effective amount of an aromatic-cationic peptide may be defined as a concentration of peptide at the target tissue of 10⁻¹² to 10⁻⁶ molar, *e.g*., approximately 10⁻⁷ molar. This concentration may be delivered by systemic doses of 0.01 to 100 mg/kg or equivalent dose by body surface area. The schedule of doses would be optimized to maintain the therapeutic concentration at the target tissue, most preferably by single daily or weekly administration, but also including continuous administration (*e.g.,* parenteral infusion or transdermal application).

In some embodiments, the dosage of the aromatic-cationic peptide is provided at about 0.001 to about 0.5 mg/kg/h, suitably from about 0.01 to about 0.1 mg/kg/h. In one embodiment, the dose of the aromatic-cationic peptide is provided from about 0.1 to about 1.0 mg/kg/h, suitably from about 0.1 to about 0.5 mg/kg/h. In one embodiment, the dose is provided from about 0.5 to about 10 mg/kg/h, suitably from about 0.5 to about 2 mg/kg/h.

The skilled artisan will appreciate that certain factors may influence the dosage and timing required to effectively treat a subject, including but not limited to, the severity of the disease or disorder, previous treatments, the general health and/or age of the subject, and other diseases present. Moreover, treatment of a subject with a therapeutically effective amount of the therapeutic compositions described herein can include a single treatment or a series of treatments.

The mammal treated in accordance present methods can be any mammal, including, for example, farm animals, such as sheep, pigs, cows, and horses; pet animals, such as dogs and cats; laboratory animals, such as rats, mice and rabbits. In one embodiment, the mammal is a human.

### Combination Therapy

In some embodiments, aromatic-cationic peptides may be combined with one or more additional therapeutic agents for the prevention, amelioration or treatment of a medical disease or condition. By way of example, but not by way of limitation, the treatment for mitochondrial diseases or disorders typically involves taking vitamins and cofactors. In addition, antibiotics, hormones, antineoplastic agents, steroids, immunomodulators, dermatologic drugs, antithrombotic, antianemic, and cardiovascular agents, by way of non-limiting example, may also be administered.

In one embodiment, aromatic-cationic peptides are combined with one or more cofactors, vitamins, iron chelators, antioxidants, frataxin level modifiers, ACE inhibitors and β-blockers. By way of example, but not by way of limitation, such compounds may include one or more of CoQ10, Levocarnitine, riboflavin, acetyl-L-carnitine, thiamine, nicotinamide, vitamin E, vitamin C, lipoic acid, selenium, β-carotene, biotin, folic acid, calcium, magnesium, phosphorous, succinate, selenium, creatine, uridine, citratesm prednisone, vitamin K, deferoxamine, deferiprone, idebenone, erythropoietin, 17β-estradiol, methylene blue, and histone deacetylase inhibitors such as BML-210 and compound 106.

In one embodiment, an additional therapeutic agent is administered to a subject in combination with an aromatic-cationic peptide, such that a synergistic therapeutic effect is produced. A "synergistic therapeutic effect" refers to a greater-than-additive therapeutic effect which is produced by a combination of at least two therapeutic agents, and which exceeds that which would otherwise result from the sole administration of the at least two therapeutic agents. Therefore, lower doses of one or more of the at least two therapeutic agents may be used in treating a disease or disorder, resulting in increased therapeutic efficacy and decreased side-effects.

The multiple therapeutic agents (including aromatic-cationic peptides) may be administered in any order or even simultaneously. If simultaneously, the multiple therapeutic agents may be provided in a single, unified form, or in multiple forms (by way of example only, either as a single pill or as two separate pills). One of the therapeutic agents may be given in multiple doses, or both may be given as multiple doses. If not simultaneous, the timing between the multiple doses may vary from more than zero weeks to less than four weeks. In addition, the combination methods, compositions and formulations are not to be limited to the use of only two agents.

### EXAMPLES

The present technology is further illustrated by the following examples, which should not be construed as limiting in any way.

### General Methods

*Reagents:* Peptides were supplied by Stealth Peptides Inc. (Newton Centre, MA). 1-palmitoyl-2-oleoyl-sn-glycero-3-phosphocholine (POPC), and cholesterol were purchased from Avanti Polar Lipids Inc. (Alabaster, AL). Amplex Red Peroxidase kit and Hepes buffer were purchased from Invitrogen (Carlsbad, CA). Cytochrome c and ethanol reagent grade ≥99.5% were purchased from Sigma (St. Louis, MO).

*Measurement of cytochrome c Peroxidase Activity:* Cytochrome c peroxidase activity was measured using the Amplex Red Peroxidase Kit. Amplex Red reagent reacts with H₂O₂ in a 1:1 ratio in the presence of a peroxidase in order to create a highly fluorescent compound called resorufin, which has an excitation at 570 nm and an emission at 585 nm. 2 µM cytochrome c was incubated in 20 mM Hepes buffer pH 7.4 with varying concentrations of free cholesterol dissolved in ethanol or cholesterol-POPC liposomes (chol-POPC). 10 µM of H₂O₂ and 50 µM of Amplex Red reagent were subsequently added. The fluorescence was then measured using time course data collected from a Spectra Max 190 microplate spectrofluorometer (Molecular Devices, Sunnyvale, CA).

*Preparation of Liposomes:* Liposomes were prepared as previously described in Melzak et al., Langmuir, 24: 9172-9180 (2008). Chol-POPC liposomes were prepared in a 1:1 molar ratio. Stock solutions were mixed and then dried under nitrogen from 30 minutes. After resuspension in 20 mM Hepes buffer pH 7.4, the liposomes were extruded 25 times through a 50 nm pore membrane (Avestin).

*Cytochrome c Reduction:* 20 µM cytochrome c in Hepes buffer pH 7.4 was contacted with varying concentrations of chol-POPC liposomes and peptide. 50 µM ascorbate (vitamin C) was added after five minutes in order to promote the reduction the cytochrome c protein. The absorbance at 550 nm was then recorded in order to obtain the rate of the reaction after treatment with varying concentrations of peptide and cholesterol using a spectrophotometer (Spectramax Gemini XPS, Molecular Devices, Sunnyvale, CA). (See Basova *et al.,* 2007).

*Circular Dichroism Spectroscopy:* The circular dichroism (CD) spectra was collected using an AVIA 62 DS spectrophotometer. The CD spectra was analyzed between 375-450 nm in order to monitor the Soret region using a CD 10-mm path-length for cells containing 20 mM Hepes (pH 7.4) and 10 mM cytochrome c in the presence or absence of 40 µM chol-POPC liposomes and/or 100 µM SS-31.

*Absorbance Spectrum:* The effects of cholesterol and aromatic-cationic peptides on the structure of cytochrome c were analyzed using absorbance spectra (Pinherio *et al.,* 1997). Wild-type cytochrome c typically has a peak at around 410 nm (which represents the Soret region) that may be affected by protein damage. Absorbance between 350-450 nm was analyzed using a Spectramax Gemini XPS spectrophotometer (Molecular Devices, Sunnyvale, CA).

*Interaction of Aromatic-cationic Peptides with Cholesterol-Cytochrome c Complex:* D-Arg-2',6'-Dmt-Lys-Ald-NH₂ ([ald]SS-31) is an aladan (ald) containing derivative of D-Arg-2',6'-Dmt-Lys-Phe-NH₂ (SS-31). *See* Birk et al., J Am Soc Nephrol. 24(8): 1250-1261 (2013). Ald, a polarity-sensitive fluorescent amino acid, increases emission intensity as the polarity of its environment decreases, and its emission maximum (λₘₐₓ) undergoes a blue shift. Thus, interaction between D-Arg-2',6'-Dmt-Lys-Ald-NH₂ ([ald]SS-31) and phospholipids causes an observable shift in the fluorescence spectra of the peptide. *See* Birk et al., J Am Soc Nephrol. 24(8): 1250-1261 (2013). The fluorescence spectra of D-Arg-2',6'-Dmt-Lys-Ald-NH₂ ([ald]SS-31) between 390 and 600 nm was analyzed to assess the interaction between the peptide and the cholesterol-cytochrome c complex.

### Example 1. Cholesterol Dose-dependently Increases Cytochrome c Peroxidase Activity.

The Amplex Red Peroxidase Kit was used to determine whether cholesterol has an effect on the peroxidase activity of cytochrome c. Cytochrome c was incubated with Amplex Red Reagent, hydrogen peroxide (H₂O₂), and varying concentrations of free cholesterol. As shown in FIG. 1, cholesterol increased cytochrome c peroxidase activity in a dose-dependent manner. For instance, incubation of cytochrome c with 300 µM free cholesterol resulted in a 40-fold increase in cytochrome c peroxidase activity. See FIG. 1.

Similar effects were observed with chol-POPC liposomes. According to FIG. 1, incubation of cytochrome c with 300 µM chol-POPC liposomes resulted in a 48-fold increase in cytochrome c peroxidase activity. This suggests that cholesterol-induced upregulation of cytochrome c peroxidase activity is not specific to free cholesterol, but is also observed with cholesterol that is associated with lipid membranes.

These results demonstrate cholesterol can impair mitochondrial function by increasing cytochrome c peroxidase activity.

### Example 2. Aromatic-cationic Peptides Inhibit Cholesterol-induced Cytochrome c Peroxidase Activity in a Dose-dependent Manner.

Cytochrome c was incubated with D-Arg-2',6'-Dmt-Lys-Phe-NH₂ (SS-31) peptide or Phe-D-Arg-Phe-Lys-NH₂ (SS-20) peptide prior to being treated with chol-POPC liposomes. As shown in FIG. 2, the aromatic-cationic peptides inhibited cholesterol-induced cytochrome c peroxidase activity in a dose-dependent manner. Cytochrome c exposed to 300 µM chol-POPC liposomes and D-Arg-2',6'-Dmt-Lys-Phe-NH₂ (SS-31) showed a 75% reduction in cytochrome c peroxidase activity compared to cytochrome c that was solely exposed to chol-POPC liposomes. Likewise, cytochrome c exposed to 300 µM chol-POPC liposomes and Phe-D-Arg-Phe-Lys-NH₂ (SS-20) showed a 77% reduction in cytochrome c peroxidase activity compared to cytochrome c that was exposed to chol-POPC liposomes only.

These results demonstrate that aromatic-cationic peptides, such as D-Arg-2',6'-Dmt-Lys-Phe-NH₂ (SS-31) or Phe-D-Arg-Phe-Lys-NH₂ (SS-20), can inhibit cholesterol-induced cytochrome c peroxidase activity. As such, these results demonstrate that the aromatic-cationic peptides of the present technology are useful in methods for reducing cholesterol-induced cytochrome c peroxidase activity in a subject suffering from a disease characterized by cholesterol-induced mitochondrial dysfunction.

### Example 3. Aromatic-cationic Peptides Mitigate the Effect of Cholesterol on Cytochrome c Reduction.

Cytochrome c must undergo reduction in order to function as an electron carrier. Impaired cytochrome c reduction contributes to mitochondrial dysfunction *via* the accumulation of ROS and peroxidized lipids. Cytochrome c is less likely to be reduced when it is damaged. (Basova *et al.,* 2007).

Cytochrome c reduction was measured in the presence of 50 µM vitamin C (an antioxidant that reduces cytochrome c), with or without chol-POPC liposomes. FIG. 3A demonstrates that cytochrome c exposed to cholesterol is less likely to be reduced compared to cytochrome c that is not incubated with cholesterol. According to FIG. 3A, cholesterol inhibited cytochrome c reduction in a dose-dependent manner. For instance, incubation of cytochrome c with 300 µM chol-POPC liposomes resulted in a 21% decline in cytochrome c reduction. See FIG. 3A. Cholesterol-induced impairment of cytochrome c reduction was reversed by the addition of either D-Arg-2',6'-Dmt-Lys-Phe-NH₂ (SS-31) peptide or Phe-D-Arg-Phe-Lys-NH₂ (SS-20) peptide. See FIG. 3B.

These results demonstrate that aromatic-cationic peptides, such as D-Arg-2',6'-Dmt-Lys-Phe-NH₂ (SS-31) or Phe-D-Arg-Phe-Lys-NH2 (SS-20), can mitigate the effect of cholesterol on cytochrome c reduction. As such, these results demonstrate that the aromatic-cationic peptides of the present technology are useful in methods for ameliorating the inhibitory effects of cholesterol on cytochrome c reduction in a subject suffering from a disease characterized by cholesterol-induced mitochondrial dysfunction.

### Example 4. The D-Arg-2',6'-Dmt-Lys-Phe-NH₂ (SS-31) Peptide Inhibits Cholesterol-induced Structural Changes in Cytochrome c.

Circular dichroism (Olis DSM20 spectropolarimeter) was carried out to analyze Soret region (375-450 nm), a probe for the attachment of the Met80 residue to the heme iron. The decreased intensity of the 416 nm Cotton effect (negative Cotton peak) is indicative of a perturbed heme pocket region, as the 416 nm dichroic band serves as a readout for the Met80-Fe(III) coordination in native cytochrome c. (Santucci *et al.,* 1997). As shown in FIG. 4, introduction of 40 µM chol-POPC liposomes induced structural changes in cytochrome c. Specifically, the negative Cotton peak at 416 nm was significantly decreased, which reveals the absence of the Met80-Fe(III) coordination. These data suggest that contact with cholesterol results in the structural alteration of cytochrome c and exposure of the heme iron. The CD spectra data comports with the findings of Examples 1 and 3 because the disruption of the Fe-Met80 coordination increases the exposure of the heme Fe to H₂O₂, thereby causing cytochrome c to switch from an electron carrier to a peroxidase.

FIG. 4 also shows that treatment with 100 µM D-Arg-2',6'-Dmt-Lys-Phe-NH₂ (SS-31) restores the negative Cotton peak of cytochrome c incubated with chol-POPC liposomes. These results demonstrate that aromatic-cationic peptides, such as D-Arg-2',6'-Dmt-Lys-Phe-NH₂ (SS-31), are capable of restoring cytochrome c structure and protecting cytochrome c from cholesterol-induced structural damage.

Cholesterol-induced structural alterations of cytochrome c were also analyzed using absorption spectroscopy. The absorbance spectrum between 350 and 450 nm was analyzed so as to monitor changes to the Soret band. A Soret band with a maximum intensity at 410 nm is indicative of the presence of both axial ligands (His18 and Met80 ligated to heme iron). (Pinherio *et al.,* 1997). As shown in FIG. 5A, incubation with chol-POPC liposomes led to a substantial reduction in the intensity of the peak at 410 nm, which is indicative of damage or alterations to the structure of cytochrome c. FIG. 5B shows that treatment with 100 µM D-Arg-2',6'-Dmt-Lys-Phe-NH₂ (SS-31) increases the intensity of the 410 nm peak of cytochrome c incubated with chol-POPC liposomes. These results demonstrate that aromatic-cationic peptides, such as D-Arg-2',6'-Dmt-Lys-Phe-NH₂ (SS-31), are capable of restoring cytochrome c structure and protecting cytochrome c from cholesterol-induced structural damage. As such, these results demonstrate that the aromatic-cationic peptides of the present technology are useful in methods for protecting cytochrome c from cholesterol-induced structural damage in a subject suffering from a disease characterized by cholesterol-induced mitochondrial dysfunction.

### Example 5. Interaction between Aromatic-Cationic Peptides and Cholesterol-Cytochrome c Complex.

The interaction between the aromatic-cationic peptide D-Arg-2',6'-Dmt-Lys-Ald-NH₂ ([ald]SS-31) and the cholesterol-cytochrome c complex was investigated using emission spectral techniques described in Birk *et al.,* 2013. As shown in FIG. 6, D-Arg-2',6'-Dmt-Lys-Ald-NH₂ ([ald]SS-31) exhibited a blue shift when it encountered 100 µM cholesterol, *i.e.,* the λₘₐₓ shifted from 530 nm to 436 nm. D-Arg-2',6'-Dmt-Lys-Ald-NH₂ ([ald]SS-31) exhibited no shift it when it contacted 4 µM cytochrome c, *i.e.,* the λₘₐₓ remained at 530 nm. These observations are consistent with the findings in Birk *et al.,* 2013.

Birk *et al.,* 2013 also demonstrated that D-Arg-2',6'-Dmt-Lys-Ald-NH₂ ([ald]SS-31) normally exhibits a smaller blue shift when it interacts with a lipid-cytochrome c complex. However, as shown in FIG. 6, D-Arg-2',6'-Dmt-Lys-Ald-NH₂ ([ald]SS-31) exhibited no shift when it contacted both cholesterol and cytochrome c. These results demonstrate that D-Arg-2',6'-Dmt-Lys-Ald-NH₂ ([ald]SS-31) effectively blocks the formation of cholesterol-cytochrome c complexes.

Taken together, these results show that aromatic-cationic peptides, such as D-Arg-2',6'-Dmt-Lys-Ald-NH₂ ([ald]SS-31), protect the structural integrity of cytochrome c against cholesterol-induced damage by inhibiting the formation of cholesterol-cytochrome c complexes. As such, these results demonstrate that the aromatic-cationic peptides of the present technology are useful in methods for inhibiting the formation of cholesterol-cytochrome c complexes in a subject suffering from a disease characterized by cholesterol-induced mitochondrial dysfunction.

Furthermore, these results show that aromatic-cationic peptides of the present technology are useful in methods for inhibiting cholesterol-induced lipid peroxidation by disrupting the interaction between cytochrome c and cholesterol, and are thus useful in methods for reducing cardiolipin oxidation in a subject suffering from a disease characterized by cholesterol-induced mitochondrial dysfunction. As such, these results show that aromatic-cationic peptides of the present technology are useful in methods for treating a subject suffering from a disease characterized by cholesterol-induced mitochondrial dysfunction.

### REFERENCES

Basova, LV, Kurnikov, IV, Kagan, VE, Bayir, ., Wang, L, Ritov, VB, et al. Cardiolipin Switch in Mitochondria: Shutting off the Reduction of Cytochrome c and Turning on the Peroxidase Activity. Biochemistry, 46(11), 3423-3434 (2007).
Birk AV, Liu S, Soon Yi, Mills W, Singh P, Warren JD, Seshan SV, Pardee JD, Szeto HH. The Mitochondrial-Targeted Compound SS-31 Re-Energizes Ischemic Mitochondria by Interacting with Cardiolipin. Journal of the American Society of Nephrology, 24(8), 1250-1261 (2013).
Borchman D, Cenedella RJ, Lamba OP. Role of Cholesterol in Structural Order of Lens Membrane Lipids. Experimental Eye Research, 62(2), 191-198 (1996).
Bosch M, Mari M, Herms A, Fernandez A, Fajardo A, Kassan A, Giralt A, Colell A, Balgoma D, Barbero E, Gonzeles-Moreno E, Matias N, Tebar F, Balsinde J, Camps M, Enrich C, Gross SP, Garcia-Ruiz C, Perez-Navarro E et al. Caveolin-1 Deficiency Causes Cholesterol-dependent Mitochondrial Dysfunction and Apoptotic Susceptibility. Current Biology, 21(8), 681-686 (2011).
Colell A, Garcia-Ruiz C, Lluis JM, Coll O, Mari M, Fernandez-Checa JC. Cholesterol Impairs the Adenine Nucleotide Translocator-mediated Mitochondrial Permeability Transition through Altered Membrane Fluidity. Journal of Biological Chemistry, 278(36), 33928-33935 (2003).
Colell A, Fernandez-Checa JC, Fernandez A. Mitochondria, Cholesterol and Amyloid β Peptide: A Dangerous Trio in Alzheimer Disease. Journal of Bioengineering, 41(5), 417-423 (2009).
Coll O, Colell A, Garcia-Ruiz C, Kaplowitz N, Fernandez-Checa JC. Sensitivity of the 2-Oxoglutarate Carrier to Alcohol Intake Contributes to Mitochondrial Glutathione Depletion. Hepatology. 38:692-702 (2003).
Echegoyen S, Olivia E, Sepulveda J, Diaz-Zagoya JC, Espinosa-Garcia T, Pardo JP, Martinez F. Cholesterol Increase in Mitochondria: Its Effect on Innermembrane Functions, Submitochondrial Localization and Ultrastructural Morphology. Journal of Biological Chemistry, 289(3), 703-708 (1993).
Fernandez A, Llacuna L, Fernandez-Checa JC, Colell A. Mitochondrial Cholesterol Loading Exacerbates Amyloid β Peptide-Induced Inflammation and Neurotoxicity. Journal of Neuroscience, 29(20), 6394-6405 (2009).
Garcia-Ruiz C, Mari M, Colell A, Caballero F, Montero J, Terrones O, Basanez G, Fernandez-Checa JC. Mitochondrial Cholesterol in Health and Disease. Histology and Histopathology, 24(1), 117-132 (2009).
Ikonen E (2008). Cellular Cholesterol Trafficking and Compartmentalization. Nature, 9(2), 125-138 (2008).
Kiebish, MA, Han, X, Cheng, H, Chuang, JH, Seyfried, TN. Cardiolipin and Electron Transport Chain Abnormalities in Mouse Brain Tumor Mitochondria: Lipidomic Evidence Supporting the Warburg Theory of Cancer. J Lipid Res 49(12): 2545-2556 (2008).
Tuominen EKJ, Wallace CJA and Kinnunen PKJ. Phospholipid-cytochrome c Interaction: Evidence for the Extended Lipid Anchorage. J Biol Chem 277:8822-8826 (2002).
Kalanxhi E and Wallace CJA. Cytochrome c Impaled: Investigation of the Extended Lipid Anchorage of a Soluble Protein to Mitochondrial Membrane Models. Biochem J 407:179-187 (2007).
Sinabaldi F, Howes BD, Piro MC, Polticelli F, Bombelli C, Ferri T et al. Extended Cardiolipin Anchorage to Cytochrome c: A Model for Protein-mitochondrial Membrane Binding. J Biol Inorg Chem 15:689-700 (2010).
Sinabaldi F, Fiorucci L, Patriarca A, Lauceri R, Ferri T, Coletta M, Santucci R. Insights into Cytochrome c -cardiolipin Interaction. Role Played by Ionic Strength. Biochemistry 47:6928-6935 (2008).
Kagan VE, Bayir A, Bayir H, Stoyanovsky D, et al. Mitochondria-targeted Disruptors and Inhibitors of Cytochrome c/cardiolipin Peroxidase Complexes. Mol Nutr Food Res 53:104-114 (2009).
Melzak KA, Bender F, Tsortos A, Electra G. Probing Mechanical Properties of Liposomes Using Acoustic Sensors. Langmuir, 24(16), 9172-9180 (2008).
Mannella C. Structure and Dynamics of Mitochondrial Inner Membrane Cristae. Biochimica et Biophysica Acta, 1763(5-6), 542-548 (2006).
Maxfield FR, Tabas I. Role of Cholesterol and Lipid Organization in Disease. Nature, 438(1), 612-621 (2005).
Mei S, Haihua G, Yang X, Guo H, Liu Z, Cao W. Prolonged Exposure to Insulin Induces Mitochondrion-Derived Oxidative Stress Through Increasing Mitochondrial Cholesterol Content in Hepatocytes. Endocrinology, 153(5), 2120-2129 (2012).
Montero J, Montserrat M, Colell A, Moralesd A, Basanez G, Garcia-Ruiz C, Fernandez-Checa J. Cholesterol and Peroxidized Cardiolipin in Mitochondrial Membrane Properties, Permeabilization and Cell Death. Biochim Biophys Acta, 1797(6-7) 1217-1224 (2010).
Montero J, Morales A, Llacuna L, Lluis JM, Terrones O, Gorka B, Antonsson B, Prieto J, Garcia-Ruiz C, Colell A, Fernandez-Checa JC. Mitochondrial Cholesterol Contributes to Chemotherapy Resistance in Hepatocellular Carcinoma. Cancer Research, 68(13), 5246-5256 (2008).
Musso G, Cambino R, Cassader M. Cholesterol Metabolism and the Pathogenesis of Non-alcoholic Steatohepatitis. Lipid Research, 52(1), 175-191 (2013).
Ning Y, Bai Q, Lu H, Li X, Pandak WM, Zhao F, Chen S, Ren S, Yin L. Overexpression of Mitochondrial Cholesterol Delivery Protein StAR, Decreases Intracellular Lipids and Inflammatory Factors Secretion in Macrophages. Atherosclerosis, 204(1), 114-120 (2009).
Paradies G, Peterosillo G, Pistolese M, Ruggiero FM. The Effect of Reactive Oxygen Species Generated from the Mitochondrial Electron Transport Chain on the Cytochrome c Oxidase Activity and on the Cardiolipin Content in Bovine Heart Submitochondrial Particles. FEBS Lett, 466(2-3), 323-326 (2000).
Paradies G, Peterosillo G, Pistolese M, Ruggiero FM. Reactive Oxygen Species Generated by the Mitochondrial Respiratory Chain Affect the Complex III Activity via Cardiolipin Peroxidation in Beef-heart Submitochondrial Particles. Mitochondrion, 1(2) 151-159 (2001).
Paradies G, Peterosillo G, Pistolese M, Ruggiero FM. Reactive Oxygen Species Affect Mitochondrial Electron Transport Complex I Activity through Oxidative Cardiolipin Damage. Gene, 286(1), 135-141 (2002).
Paradies G, Petrosillo S, Paradies V, Ruggiero FM. Role of Cardiolipin Peroxidation and Ca2+ in Mitochondrial Dysfunction and Disease. Cell Calcium, 45(6), 643-650 (2009).
Pinherio JT, Elove GA, Watts A, Roder H. Structural and Kinetic Description of Cytochrome c Unfolding Induced by Interaction with Lipid Vesicles. Biochemistry, 36(42), 13122-12132 (1997).
Pierce MM, Nall BT. Fast Folding of Cytochrome C. Protein Science, 6(3), 618-627 (1997).
Reynolds GA, Basu SK, Osborne TF, Chin DJ, Gil G, Brown MS, Goldstein JL, Luskey KL. HMG CoA reductase: A Negatively Regulated Gene with Unusual Promoter and 5' Untranslated Regions. Cell, 38(1), 275-285 (1984).
Rogers KS, Higgins ES, Grogan WM. Influence of Dietary Cholesterol on Mitochondrial Function in the Rat. Journal of Nutrition, 110(2), 248-254 (1979).
Rouslin W, MacGee J, Gupte S, Wesselman A, Epps DE. Mitochondrial Cholesterol Content and Membrane Properties in Porcine Myocardial Ischemia. American Journal of Physiology- Heart and Circulatory Physiology, 242(2), 254-259 (1982).
Santucci R, Ascoli F. The Soret Circular Dichroism Spectrum as a Probe for the Heme Fe(III)-Met(80) Axial Bond in Horse Cytochrome c. J Inorg Biochem, 68(3), 211-214 (1997).
Schlame M, Rua D, Greenberg ML. The Biosynthesis and Functional Role of Cardiolipin. Prag Lipid Res, 39(3). 257-288 (2002).
Szeto HH, Schiller PW. Novel Therapies Targeting Inner Mitochondrial Membrane From Discovery to Clinical Development. Pharm Res, 28(11), 2669-2679 (2011).
Szeto HH, Liu S, Soong Y, Wu D, Darrah SF, Chen FY, Zhao Z, Granger M, Tow CY, Seshan SV. Mitochondria-Targeted Peptide Accelerates ATP Recovery and Reduces Ischemic Kidney Injury. Journal of American Society of Nephrology, 22(6), 1041-1052 (2011).
Unsay J, Cosentino K, Yamunadevi S, Garcia-Saez A. Cardiolipin Effects on Membrane Structure and Dynamics. Langmuir, (2013) (manuscript accepted).
Van Meer G, Voelker DR, Feigenson GW. Membrane Lipids: Where they are and how they Behave. Nat Rev Molecular Cellular Biology, 9(2), 112-124 (2008).
Vladimirov YA, Proskurnina EV, Izmailov DY, Novikov AA, Brusnichkin AV, Osipov AN, Kagan VE. Mechanism of Activation of Cytochrome c Peroxidase Activity by Cardiolipin. Biochemistry, 71 (9), 989-997 (2006).
Yu W, Gong J, Mihee K, Garver WS, Yangisawa K, Michikawa M. Altered Cholesterol Metabolism in Niemann-Pick Type Cl Mouse Brain Affects Mitochondrial Function. Journal of Biological Chemistry, 280(12), 11731-11739 (2005).
Zager RA, Johnson A, Anderson K, Wright S. Cholesterol Ester Accumulation: An Immediate Consequence of Acute in vivo Ischemic Renal Injury. Kidney International, 59(5) 1750-1761 (2001).
Zhao K, Zhao G, Wu D, Soong Y, Birk AV, Schiller PW, Hazel HH. Cell-permeable Peptide Antioxidant Targeted to Inner Mitochondrial Membrane Inhibits Mitochondrial Swelling, Oxidative Cell Death, and Reperfusion. Journal of Biological Chemistry, 279(33), 34682-34690 (2004).

### EQUIVALENTS

The present technology is not to be limited in terms of the particular embodiments described in this application, which are intended as single illustrations of individual aspects of the present technology. Many modifications and variations of this present technology can be made without departing from its scope, as will be apparent to those skilled in the art. Functionally equivalent methods and apparatuses within the scope of the present technology, in addition to those enumerated herein, will be apparent to those skilled in the art from the foregoing descriptions. Such modifications and variations are intended to fall within the scope of the appended claims. The present technology is to be limited only by the terms of the appended claims, along with the full scope of equivalents to which such claims are entitled. It is to be understood that this present technology is not limited to particular methods, reagents, compounds compositions or biological systems, which can, of course, vary. It is also to be understood that the terminology used herein is for the purpose of describing particular embodiments only, and is not intended to be limiting.

In addition, where features or aspects of the disclosure are described in terms of Markush groups, those skilled in the art will recognize that the disclosure is also thereby described in terms of any individual member or subgroup of members of the Markush group.

As will be understood by one skilled in the art, for any and all purposes, particularly in terms of providing a written description, all ranges disclosed herein also encompass any and all possible subranges and combinations of subranges thereof. Any listed range can be easily recognized as sufficiently describing and enabling the same range being broken down into at least equal halves, thirds, quarters, fifths, tenths, *etc.* As a non-limiting example, each range discussed herein can be readily broken down into a lower third, middle third and upper third, *etc.* As will also be understood by one skilled in the art all language such as "up to," "at least," "greater than," "less than," and the like, include the number recited and refer to ranges which can be subsequently broken down into subranges as discussed above. Finally, as will be understood by one skilled in the art, a range includes each individual member. Thus, for example, a group having 1-3 cells refers to groups having 1, 2, or 3 cells. Similarly, a group having 1-5 cells refers to groups having 1, 2, 3, 4, or 5 cells, and so forth.

Other embodiments are set forth within the following claims.

## Claims

1. An aromatic-cationic peptide or a pharmaceutically acceptable salt thereof for use in treating a subject suffering from Niemann-Pick Disease, wherein the aromatic cationic peptide is selected from the group consisting of 2',6'-Dmt-D-Arg-Phe-Lys-NH₂ (SS-02), Phe-D-Arg-Phe-Lys-NH₂ (SS-20), and D-Arg-2',6'-Dmt-Lys-Phe-NH₂ (SS-31), and wherein the Niemann-Pick Disease is **characterized by** cholesterol-induced mitochondrial dysfunction.

2. An aromatic-cationic peptide or a pharmaceutically acceptable salt thereof for use according to claim 1, wherein the salt is an acetate salt, tartrate salt or trifluoroacetate salt.

3. An aromatic-cationic peptide or a pharmaceutically acceptable salt thereof for use according to claim 1 wherein the peptide is D-Arg-2',6'-Dmt-Lys-Phe-NH₂ (SS-31).

4. An aromatic-cationic peptide or a pharmaceutically acceptable salt thereof for use according to claim 1, wherein treatment comprises reducing or ameliorating one or more symptoms of Niemann-Pick Disease, wherein the symptoms of Niemann-Pick Disease are one or more symptoms selected from the group consisting of: difficulty moving limbs (dystonia), enlargement of the spleen and liver (hepatosplenomegaly), dementia, slurred, irregular speech (dysarthria), discoordinated swallowing (dysphagia), sudden loss of muscle tone which may lead to falls (cataplexy), tremors, difficulties moving the eyes up and down (vertical supranuclear gaze palsy), ataxia, reduced appetite, abdominal distension, thrombocytopenia, jaundice, pain, enlarged bone marrow cavities, thinned cortical bone, distortion of the hip bone, seizures, cherry red spot in the eye, and sleep related disorders.

5. An aromatic-cationic peptide or a pharmaceutically acceptable salt thereof for use according to claim 1 , wherein the aromatic-cationic peptide is intended to be administered orally, parenterally, intravenously, subcutaneously, transdermally, topically or by inhalation.

## Patentansprüche

1. Aromatisch-kationisches Peptid oder pharmazeutisch unbedenkliches Salz davon zur Verwendung bei der Behandlung eines an Niemann-Pick-Krankheit leidenden Subjekts, wobei das aromatisch-kationische Peptid aus der aus 2',6'-Dmt-D-Arg-Phe-Lys-NH₂ (SS-02), Phe-D-Arg-Phe-Lys-NH₂ (SS-20) und D-Arg-2',6'-Dmt-Lys-Phe-NH₂ (SS-31) bestehenden Gruppe ausgewählt ist und wobei die Niemann-Pick-Krankheit durch cholesterininduzierte mitochondriale Dysfunktion gekennzeichnet ist.

2. Aromatisch-kationisches Peptid oder pharmazeutisch unbedenkliches Salz davon zur Verwendung nach Anspruch 1, wobei es sich bei dem Salz um ein Acetatsalz, Tartratsalz oder Trifluoracetatsalz handelt.

3. Aromatisch-kationisches Peptid oder pharmazeutisch unbedenkliches Salz davon zur Verwendung nach Anspruch 1, wobei es sich bei dem Peptid um D-Arg-2',6'-Dmt-Lys-Phe-NH₂ (SS-31) handelt.

4. Aromatisch-kationisches Peptid oder pharmazeutisch unbedenkliches Salz davon zur Verwendung nach Anspruch 1, wobei die Behandlung die Verminderung bzw. Verbesserung eines oder mehrerer Symptome der Niemann-Pick-Krankheit umfasst, wobei die Symptome der Niemann-Pick-Krankheit eines oder mehrere Symptome ausgewählt aus der Gruppe bestehend aus: Schwierigkeiten beim Bewegen der Gliedmaßen (Dystonie), Vergrößerung von Milz und Leber (Hepatosplenomegalie), Demenz, undeutlichem, unregelmäßigen Sprechen (Dysarthrie), Schwierigkeiten beim Schlucken (Dysphagie), einem plötzlichen Verlust der Muskelspannung, der zu Stürzen führen kann (Kataplexie), Zittern, Schwierigkeiten beim Hoch- und Runterbewegen der Augen (vertikale supranukleäre Blickparese), Ataxie, Appetitverlust, Bauchauftreibung, Thrombozytopenie, Gelbsucht, Schmerzen, vergrößerten Knochenmarkhöhlen, einer Verdünnung des Kortikalis, einer Deformation des Hüftknochens, Krampfanfälle, einem kirschroten Fleck im Auge und Schlafstörungen sind.

5. Aromatisch-kationisches Peptid oder pharmazeutisch unbedenkliches Salz davon zur Verwendung nach Anspruch 1, wobei das aromatisch-kationische Peptid für die orale, parenterale, intravenöse, subkutane, transdermale oder topische Verabreichung oder die Verabreichung durch Inhalation bestimmt ist.

## Revendications

1. Peptide cationique aromatique ou sel pharmaceutiquement acceptable de celui-ci pour utilisation dans le traitement d'un sujet souffrant de la maladie de Niemann-Pick, le peptide cationique aromatique étant choisi dans le groupe constitué de 2',6'-Dmt-D-Arg-Phe-Lys-NH₂ (SS-02), Phe-D-Arg-Phe-Lys-NH₂ (SS-20), et D-Arg-2',6'-Dmt-Lys-Phe-NH₂ (SS-31), et la maladie de Niemann-Pick étant **caractérisée par** un dysfonctionnement mitochondrial induit par le cholestérol.

2. Peptide cationique aromatique ou sel pharmaceutiquement acceptable de celui-ci pour utilisation selon la revendication 1, le sel étant un sel d'acétate, un sel de tartrate ou un sel de trifluoroacétate.

3. Peptide cationique aromatique ou sel pharmaceutiquement acceptable de celui-ci pour utilisation selon la revendication 1, le peptide étant D-Arg-2',6'-Dmt-Lys-Phe-NH₂ (SS-31).

4. Peptide cationique aromatique ou sel pharmaceutiquement acceptable de celui-ci pour utilisation selon la revendication 1, le traitement comprenant la réduction ou l'amélioration d'un ou plusieurs symptômes de la maladie de Niemann-Pick, les symptômes de la maladie de Niemann-Pick étant un ou plusieurs symptômes choisis dans le groupe constitué de : une difficulté à bouger les membres (dystonie), une hypertrophie de la rate et du foie (hépatosplénomégalie), la démence, une élocution difficile et défectueuse (dysarthrie), une déglutition (dysphagie), une perte soudaine de tonus musculaire qui peut entraîner des chutes (cataplexie), des tremblements, des difficultés à bouger les yeux vers le haut et le bas (paralysie supranucléaire verticale du regard), l'ataxie, la réduction de l'appétit, une distension abdominale, la thrombopénie, la jaunisse, une douleur, des cavités de la moelle osseuse dilatées, un amincissement de l'os cortical, une déformation de l'os de la hanche, des convulsions, une tache rouge cerise dans l'oeil et des troubles du sommeil.

5. Peptide cationique aromatique ou sel pharmaceutiquement acceptable de celui-ci pour utilisation selon la revendication 1, le peptide cationique aromatique étant destiné à être administré par voie orale, par voie parentérale, par voie intraveineuse, par voie sous-cutanée, par voie transdermique, par voie topique ou par inhalation.
